(19) Europäisches Patentamt
European Patent Office
Office européen des brevets

(11) EP 1 891 924 B1

(12) **EUROPEAN PATENT SPECIFICATION**

(45) Date of publication and mention of the grant of the patent:
**16.11.2016 Bulletin 2016/46**

(21) Application number: **06757274.3**

(22) Date of filing: **13.06.2006**

(51) Int Cl.:
***A61K 8/44*** *(2006.01)* ***A61K 31/16*** *(2006.01)*
***A61P 17/16*** *(2006.01)* ***A61Q 17/00*** *(2006.01)*
***A61Q 5/00*** *(2006.01)*

(86) International application number:
**PCT/JP2006/311789**

(87) International publication number:
**WO 2006/134890 (21.12.2006 Gazette 2006/51)**

(54) **COMPOSITION FOR PROTECTION OF BODY SURFACE**

ZUSAMMENSETZUNG ZUM SCHUTZ DER KÖRPEROBERFLÄCHE

COMPOSITION POUR PROTÉGER LA SURFACE D UN CORPS

(84) Designated Contracting States:
**DE FR GB**

(30) Priority: **14.06.2005 JP 2005173233**
**16.08.2005 JP 2005235803**

(43) Date of publication of application:
**27.02.2008 Bulletin 2008/09**

(73) Proprietor: **ASAHI KASEI KABUSHIKI KAISHA**
**Tokyo 101-8101 (JP)**

(72) Inventors:
- **KANDA, Kentarou**
**1008440 (JP)**
- **YAMAWAKI, Yukio**
**1008440 (JP)**
- **SAITOU, Yamato**
**1008440 (JP)**

(74) Representative: **Strehl Schübel-Hopf & Partner**
**Maximilianstrasse 54**
**80538 München (DE)**

(56) References cited:
**EP-A1- 1 547 998**
**JP-A- 07 187 987**
**JP-A- 11 180 844**
**JP-A- H09 124 432**
**JP-A- 2004 168 675**
**JP-A- 2004 168 735**
**JP-A- 2004 168 736**
**JP-A- 2004 331 594**
**JP-A- 2006 045 063**
**JP-A- 2006 045 064**
**JP-A- 2006 056 874**

- **TAKAGI Y. ET AL.: 'Saikin no Ceramide Kenkyu Kaihatsu no Genjo. (Recent progress on the study of artificial ceramides especially regarding the barrier function of stratum corneum)' FRAGRANCE JOURNAL 1999 NEN, 10 GATSUGO 15 October 1999, pages 9 - 16, XP003006026**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 891 924 B1

## Description

TECHNICAL FIELD

The present invention relates to a composition that is used in applications to prevent or reduce disorders on a body surface (skin, hair), specifically relates to a body surface protecting composition. In particular, the present invention relates to a body surface protecting composition which has the excellent effects of accelerating the recovery of the barrier functions of skin and hair, and preventing degradation of those functions.

BACKGROUND ART

Skin barrier functions include a function that prevent invasion by foreign matter from outside the skin into a body, and a function that prevents water content in the body from escaping to the outside. When the barrier function of the skin is degraded, rough skin or inflammation of the skin such as rash can result from external stimulation, and dry chapped skin can result from an increase in the transpiration of water from the skin, and therefore itchiness and skin trouble can easily occur. Furthermore, the barrier function of the hair is primarily a function of preventing water and aqueous components from escaping to the outside. When the barrier function of the hair is reduced, the tensile strength and viscoelasticity of the hair will be degraded, the feel on the skin will be poor, hair styling will be difficult, and not only will this induce a deterioration in appearance such as luster, but will also cause damage such as split ends and broken hair.

Conventionally moisturizing agents have been added to cosmetic materials in order to improve and prevent this type of dry condition in skin and hair. For example, patent document 1 discloses a cosmetic material for skin that contains a polyol such as glycerin or ethylene glycol, or urea, hyaluronic acid or an amino acid or the like as a moisturizing agent. However, with these cosmetic materials, the effect is temporary and is insufficient for resolving the aforementioned problems. Furthermore, this cosmetic material is not used for applications to reduce and prevent damage to the surface of the body, and there is no comment whatsoever concerning an effect of accelerating improvements in the barrier function of the skin or preventing a degradation in that function. Furthermore, according to recent dermatological research, simple application of a moisturizing agent onto the surface of skin will increase the moisture of the skin for a short period of time, but in actuality may hinder the intrinsic function of the skin to retain moisture in the skin, and is known to damage the condition of the skin.

Furthermore, patent document 2 for instance discloses a composition comprising an anionic surfactant with two chains and two hydrophilic groups, and discloses the use as a cosmetic material with moisturizing effects. However, this composition is also not used for applications to reduce and prevent damage to the surface of the body, and it dose not disclose any effect of accelerating improvements in the barrier function of the skin or preventing any degradation in that function, and does not disclose a compound that has those effects.

Furthermore, patent document 3 discloses a moisture retaining composition which does not feel sticky by the combined use of a double chain amide compound with a polyhydric alcohol. However, this moisture retaining material is essentially a polyhydric alcohol, and similar to patent document 1, is not used for applications to reduce and prevent damage to the surface of the body, and it does not disclose any effect of accelerating improvements in the barrier function of the skin or preventing a degradation in net function, and only discloses the fact that a double chain amide compound works to reduce the stickiness when the composition is applied.

Patent document 4 discloses a composition with low irritability that contains an anionic surfactant with a double chain bipolar acyl group, but it only disclose the low irritability of the composition, and dose not disclose any application for preventing or reducing damage to the surface of the body, nor any effect of proactively accelerating an improvement of the skin barrier function or preventing a degradation of that function.

Patent document 5 discloses an agent to improve skin damage and metabolic disorders using a Gemini type surfactant, but the Gemini surfactant disclosed in this document is only a carrier for transporting physiologically active materials through skin or membrane, and does not show an effect of accelerating an improvement in the skin barrier function or preventing deterioration of that function.

On the other hand, a type of sphingolipid such as ceramides obtained either naturally or synthetically are well-known as substances which improve the skin barrier function, and for example, patent document 6 discloses a liquid crystal composition using various types of ceramides, patent document 7 discloses an external medicine that contains ceramides or substances with a structure similar to a ceramide, and patent document 8 discloses a skin care agent that contains ceramides, and discloses a composition that improves skin turn over, improves skin roughness, and has excellent permeability and moisture retaining properties. However, all of the ceramides used in these inventions have low water solubility, and not only cause difficulty when creating a composition, but also require improvisations in order to permeate into the skin and hair, and cause restrictions on the degree of freedom of the formulation.

Therefore there is demand for a raw material which has a strong effect at accelerating the recovery of the barrier function of skin and hair or in other words the surface of the body, and has good water solubility.

On the other hand, in patent document 9, the present inventors have disclosed a surfactant consisting of a composition containing an acyl group comprising an N-long chain acyl acidic amino acid derivative made by reacting N-long chain acyl acidic amino acid anhydride with at least one compound selected from compounds which have m functional groups of at least one type selected from a group consisting of a hydroxyl group, an amino group, and a thiol group in the molecule, as well as a manufacturing method thereof. In this publication, the surfactant obtained provides surface activity at low concentrations and has been confirmed to have low irritability.

Patent document 1 Japanese Patent Application Laid-open No. 2000-191499
Patent document 2 Japanese Patent Application Laid-open No. H10-218754
Patent document 3 Japanese Patent Application Laid-open No. H10-203956
Patent document 4 Japanese Patent Application Laid-open No. H10-219278
Patent document 5 WO2005/39642
Patent document 6 Japanese Patent Application Laid-open No. H9-124432
Patent document 7 Japanese Patent Application Laid-open No. H5-213731
Patent document 8 Japanese Patent Application Laid-open No. 2001-39859
Patent document 9 Japanese Patent Application Laid-open No. 2002-167313 JP 2004-168735 A, EP 1547998 A1 (WO 2004/020394 A1), JP 2004-168736 A, JP 2004-168675 A, JP 2006-045063A, JP 2006-045064 A and JP 2006-056874 A disclose the addition of cholesterols as optional ingredients to pharmaceutical and/or cosmetic compositions comprising a multi-chain multiple hydrophilic group-type compound.

DISCLOSURE OF INVENTION

PROBLEMS TO BE SOLVED BY THE INVENTION

An object of the present invention is to provide a body surface protecting composition which has an excellent effect of accelerating recovery of the barrier function of skin and hair when that function has deteriorated due to skin trouble or the like.

As a result of diligent investigations, the present inventors have discovered that composition comprising a multi-chains and multiple hydrophilic group-type compound that has two or more hydrophobic groups and two or more hydrophilic groups in a molecule, as defined in claim 1, has an excellent effect of improving the barrier function of the skin and hair that is required because of skin trouble or the like, when combined with a liquid crystal forming aid as defined in claim 1.

As described above, this multi-chain multiple hydrophilic group-type compound is known to be a surfactant, but the effect of improving the barrier function of skin and hair is entirely unknown.
Furthermore, single chain single hydrophilic group-type compounds which have essentially the same basic constitutional units as multi-chain multiple hydrophilic group-type compounds can not provide the effect of accelerating recovery of the barrier function of skin, and conversely cause the barrier function to deteriorate. Therefore, it was unexpected that multi-chain multiple hydrophilic group-type compounds have the effect of improving the barrier function of skin and hair.

The effect of improving the barrier function of skin and hair with the present invention is achieved because the multi-chain multiple hydrophilic group-type compound forms a liquid crystal, and the liquid crystal formation is achieved by combining an oil-based component which is a sterol compound with the multi-chain multiple hydrophilic group-type compound in a specific molar ratio.

In other words, the present invention is a body surface protecting composition as defined in claim 1.

EFFECT OF THE INVENTION

The composition of the present invention has an excellent effect of accelerating recovery of the barrier function of skin and hair when that function has deteriorated due to skin trouble or the like. Furthermore, the composition of the present invention also has an excellent effect of restoring damaged tips of hair.

BEST MODE FOR CARRYING OUT THE INVENTION

The present invention will be described below in detail with a particular focus on working examples. (A) that is used in the body surface protecting composition of the present invention is a multi-chain multiple hydrophilic group-type compound which has two or more hydrophobic groups and two or more hydrophilic groups in a molecule and is described further below.

When the multi-chain multiple hydrophilic group-type compound of (A) is a salt, examples can include alkali metal salts, alkali earth metal salts, polyvalent metal salts, ammonium salts, organic amine salts, and basic amino acid

salts and the like. Specific examples of these salts include one or more types of salt arbitrarily selected from salt of alkali metals such as sodium, potassium, and lithium, alkali earth metals such as calcium and magnesium, metals such as aluminum, zinc, iron, cobalt, titanium, and zirconium, organic amines such as ammonia, monoethanolamine, diethanolamine, triethanolamine, and triisopropanolamine, and basic amino acids such as arginine and lysine. Of these salts, sodium salt, potassium salt, organic amine salt, and basic amino acid salt are particularly preferable.

The top layer of human skin and hair is a layer known as the horny layer, and this horny layer consits of cells that have been keratinized (keratinized cells), and "intercellular lipids" that fill in the gaps of the cells. The barrier function of the skin is thought to be primarily dependent on the "intercellular lipids", and these "intercellular lipids" are primarily made of ceramides, and align in plane to adopt a "lamellar structure" that retains moisture between the structural elements. Degradation of the barrier function of skin and hair is thought to be caused by a loss of ceramides from the horny layer for some reason, and by disorganizing of the lamellar structure of the intercellular lipids.

It is hypothesized that the multi-chain multiple hydrophilic group-type compound of the present invention can easily align in a plane because of the bulk balance and positional relationship of the hydrophobic and hydrophilic groups, and when the multi-chain multiple hydrophilic group-type compound permeates into the horny layer of skin where the intracellular lipid lamellar structure has become disorganized, the compound can easily create the lamellar structure together with the remaining ceramide. Furthermore, the surface activity and the permeability are high, so the compound easily penetrates into the horny layer when applied to the surface of the skin. Therefore, it is thought that when the multi-chain multiple hydrophilic group-type compound expressed by formula (1) is applied to the surface of the skin, the compound permeates into the horny layer, acts in a manner similar to ceramide replacing the ceramide that was lost, and restores the barrier function of the skin.

The compounds (A) of the present invention are shown below.

compound a

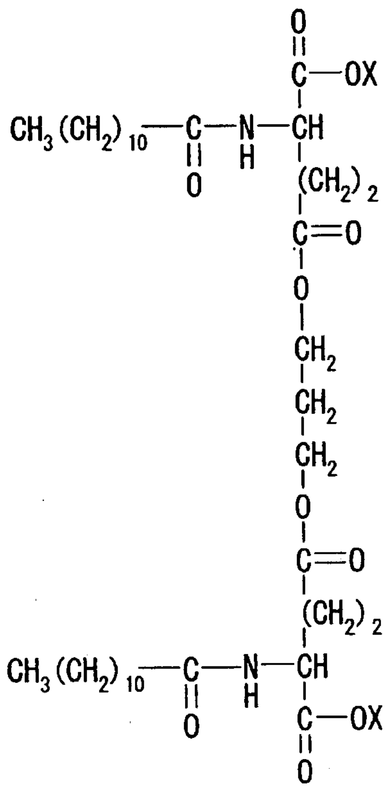

[C 8]

compound b

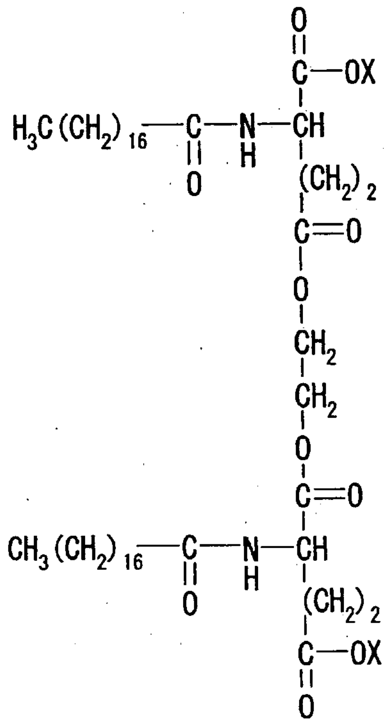

[C 9]

compound C

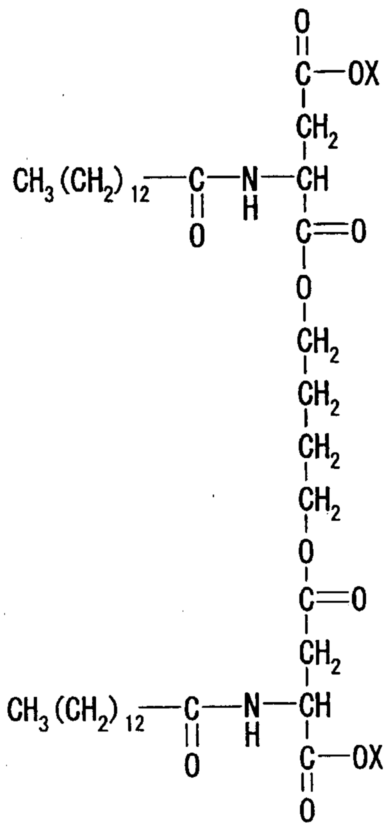

[C 10]

compound d

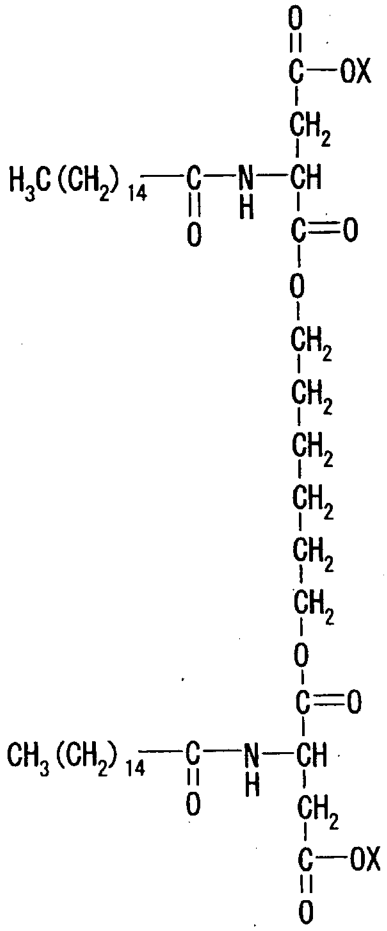

[C11]

compound e

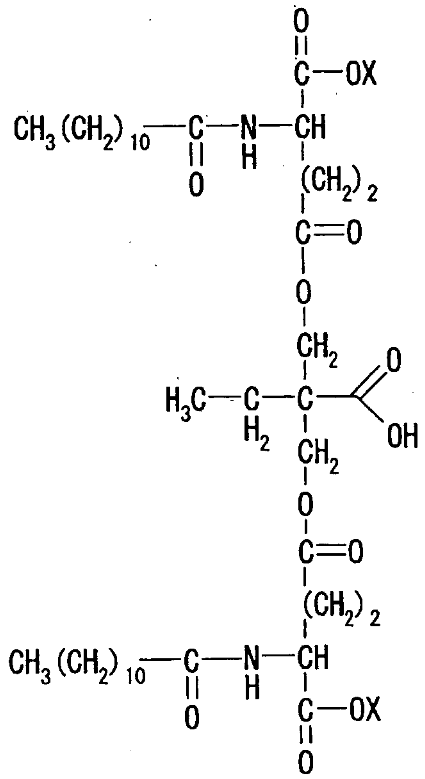

[C 12]

compound f

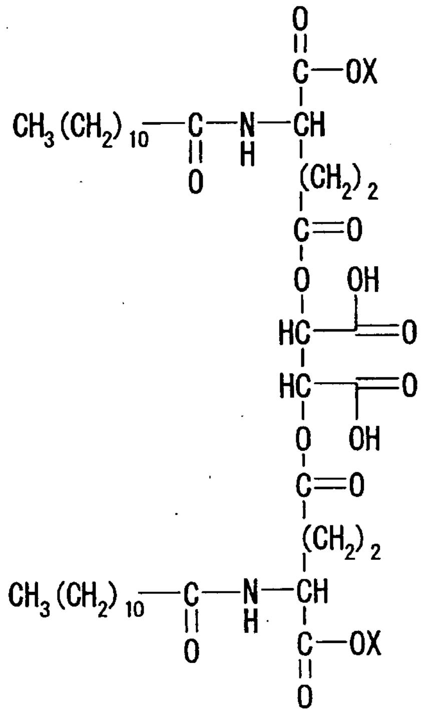

[C 13]

compound g

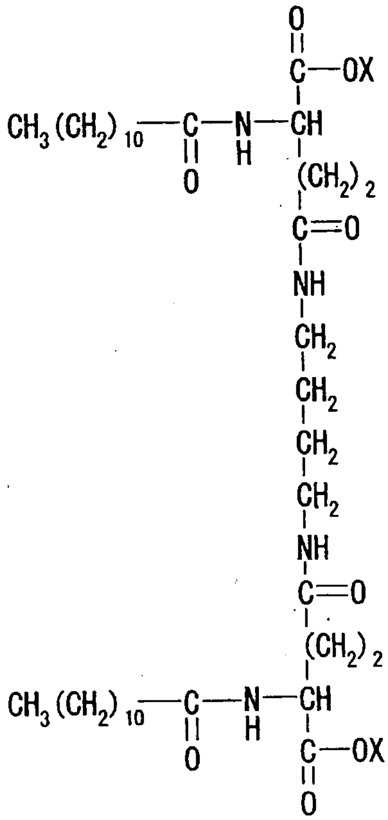

[C 14]

compound h

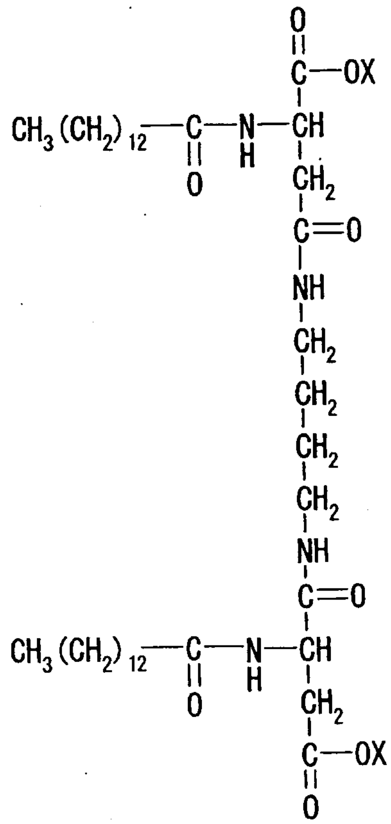

[C 15]

compound i

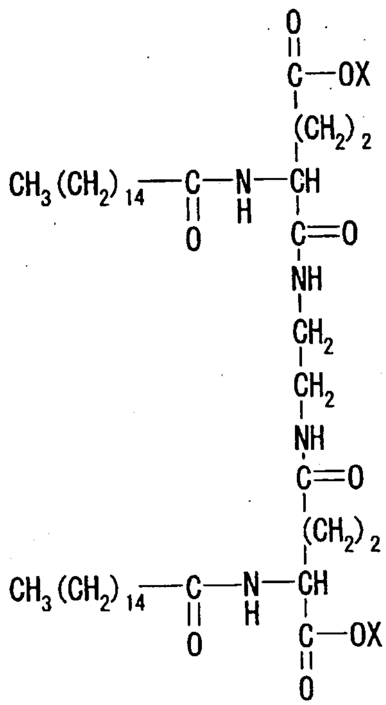

[C 16]

compound j

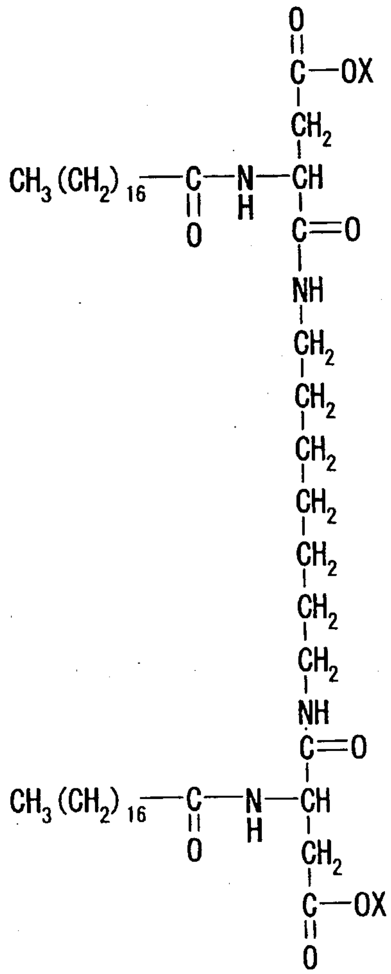

[C 17]

compound k

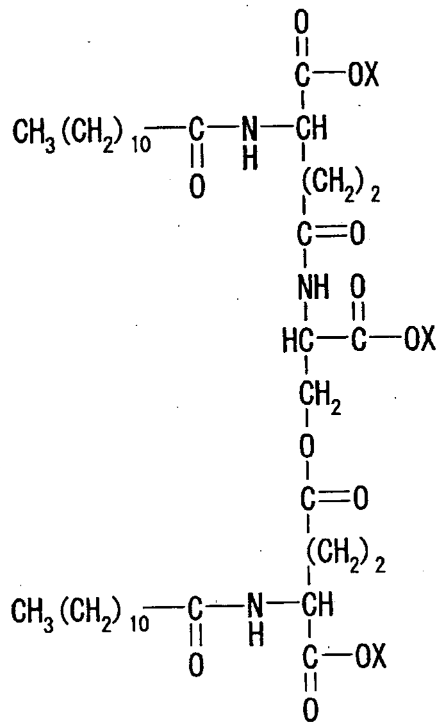

[C 18]

compound l

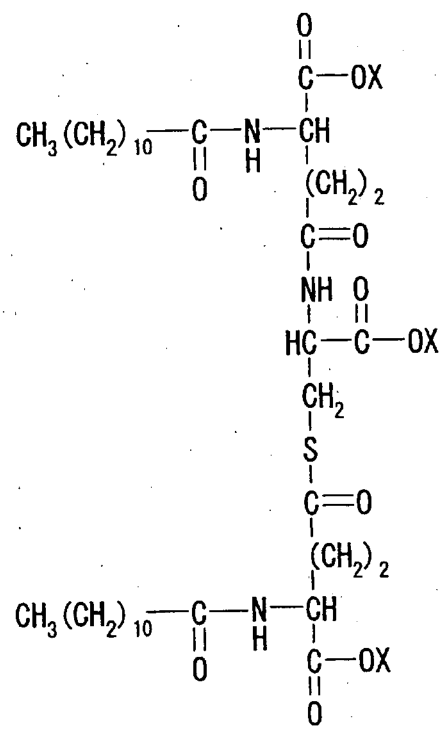

and compounds (mixtures) represented by the following formulae (5) to (7)

....(5)

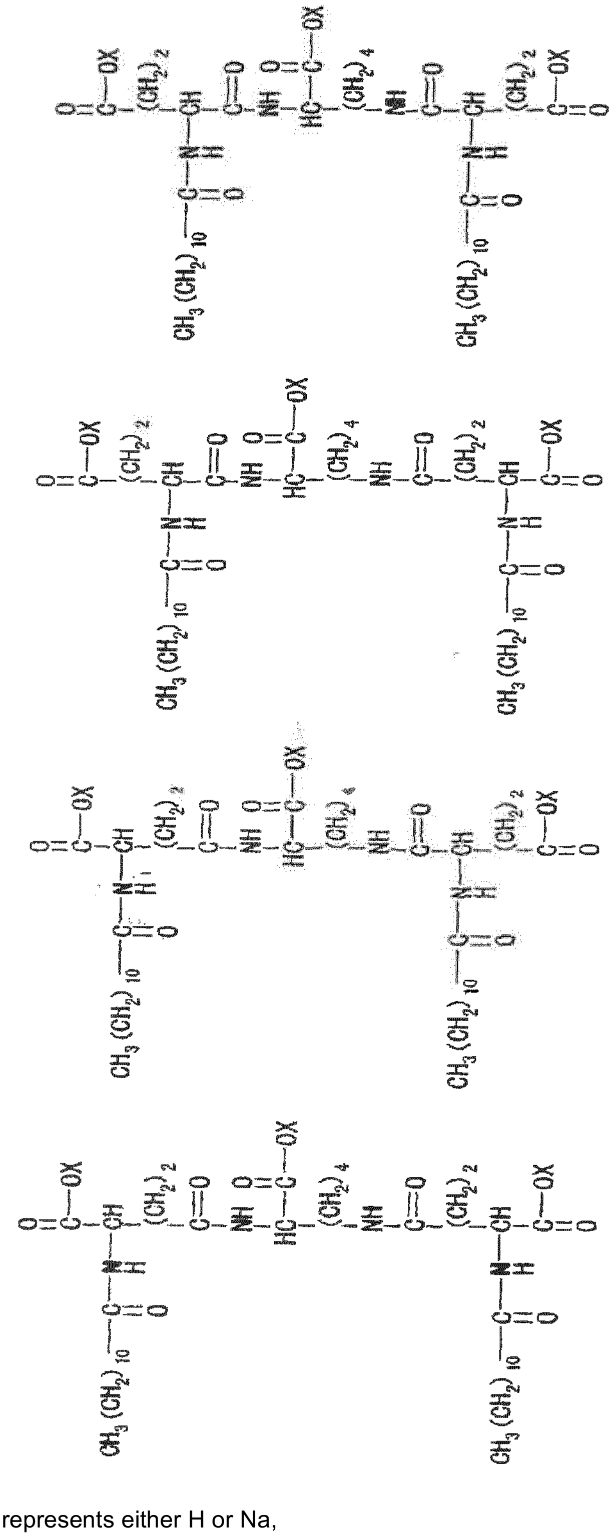

wherein X independently represents either H or Na,

...(6)

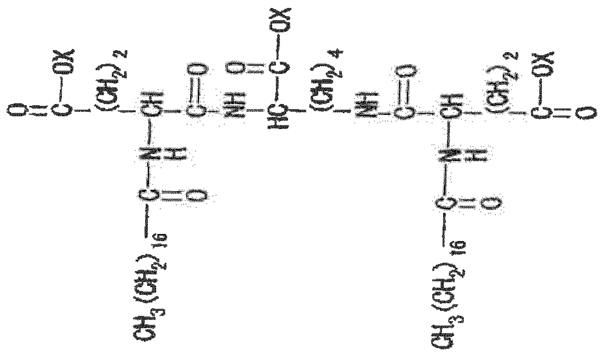

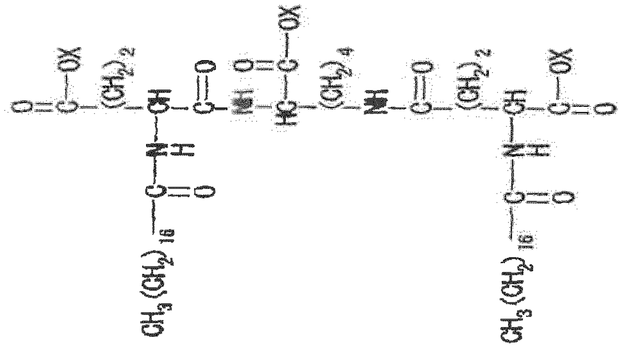

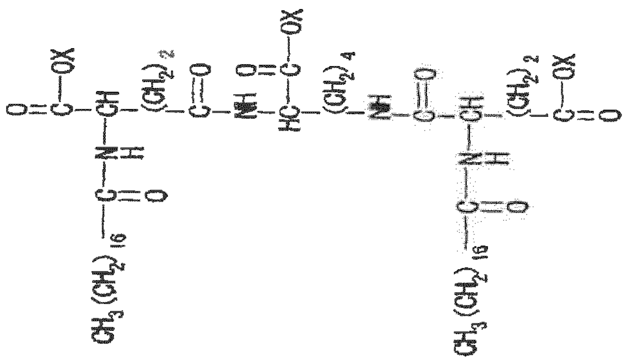

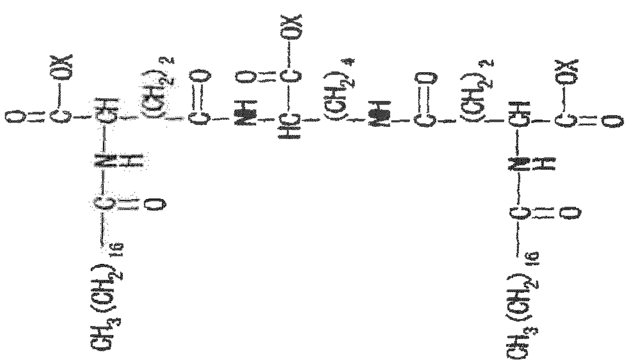

wherein X independently represents either H or Na,

···(7)

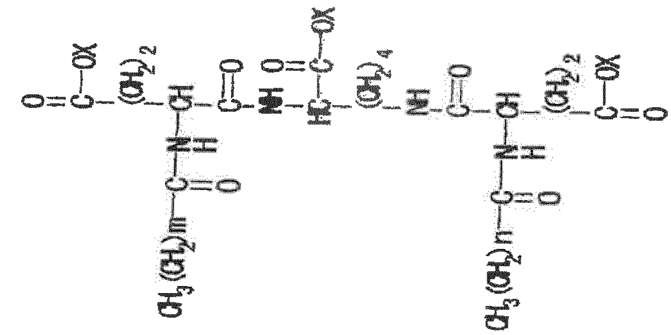

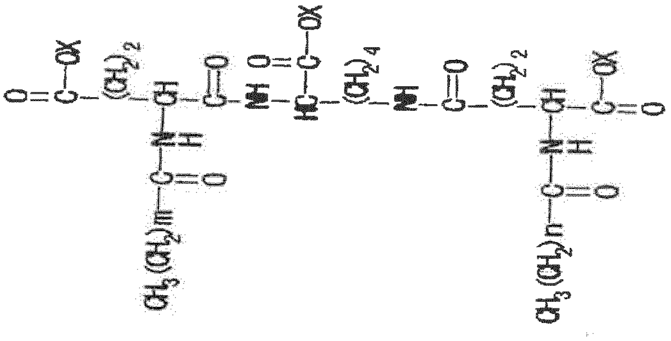

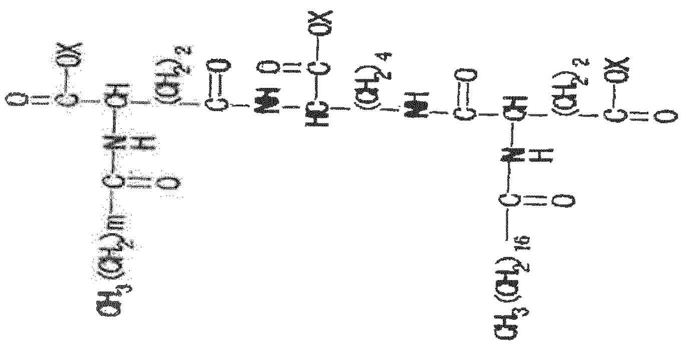

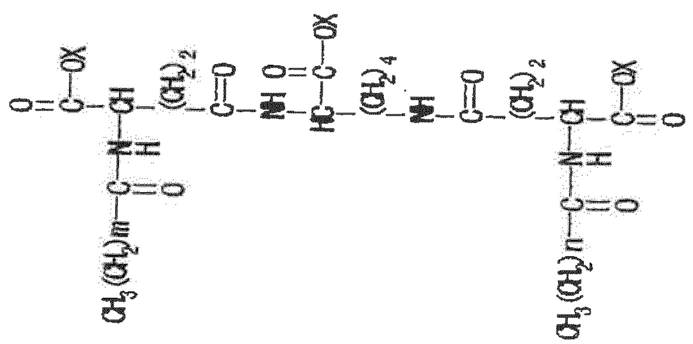

wherein X independently represents either H or Na, and m and n each independently represent integers from 6 to 16.

The amount of the multi-chain multiple hydrophilic group-type compound of (A) in the body surface protecting composition of the present invention is preferably from 0.001 to 20 mass%, more preferably from 0.01 to 10 mass%, and yet more preferably from 0.01 to 5 mass%. If the amount of multi-chain multiple hydrophilic group-type compound is below the aforementioned range, the effect of improving or preventing a degradation in the barrier function of the skin

may not be sufficient.

The oil-based component of (B) that is added in a embodiment of the body surface protecting composition of the present invention is at least one type of a sterol compound.

(B) is a liquid crystal formation aid that has excellent performance in accelerating the liquid crystal formation of (A).

Oil components which have an excellent performance at accelerating the liquid crystal formation of (A) include cholesterol esters, sterols and derivatives thereof.

Specific examples include cholesterol esters such as cholesterol palmitate, cholesterol isostearate, di(cholesterol, 2-octyldodecyl) acylglutamate, cholesterol lanolate, and cholesterol hydroxystearate; animal or plant sterols such as cholesterol and phytosterol as well as derivatives thereof.

Of these at least one type of sterol compound selected from cholesterol, cytosterol, lanosterol, phytosterol, dihydrocholesterol, dihydrolanosterol, dehydrochloresterol and esters thereof are preferable.

From the viewpoint of the effect of accelerating the liquid crystal formation of (A), the molar ratio of (B) with regards to component (A) (amount of (B): amount of (A)) is 1:10 or higher and 10:1 or lower, and more preferably the molar ratio with regards to (A) is 1:5 or higher and 5:1 or lower.

The horny layer intercellular component of (C) added to the example of the body surface protecting composition of the present invention is a water-soluble component other than (B) that exists in the horny layer intercellular space, and examples include NMF (natural moisturizing factors) such as amino acids, lactic acid, urea, and citrates.

The water-soluble moisturizing agent (D) added to the preferable embodiment of the body surface protecting composition of the present invention is a water-soluble component that has moisture retaining capability other than (C) and that essentially does not exist naturally in the horny layer intercellular space, and examples include: polyols such as glycerin, diglycerin, polyglycerin, 1,3-butanediol, propanediol, and polyethylene glycol; alkyl glycines such as N-methylglycine, N,N- dimethylglycine, N,N,N-trimethylglycine, and N-ethylglycine; (poly) saccharides and derivatives thereof such as sorbitol, raffinose, pyrrolidone carboxylates, lactate, hyaluronates, ceramides, trehalose, xylobiose, maltose, sucrose, glucose, and vegetable viscosity modifying polysaccharides; and water-soluble moisture retaining substances such as water-soluble chitin, chitosan, pectin, chondroitin sulfate, and other glycosamino glycanes and salts thereof, glycine, serine, theonine, alanine, asparatic acid, thyrosine, valine, luecine, alginine, glutamine, prolinic acid and other amino acides and salts thereof, aminocarbonyl reaction products and other saccharide amino acid compounds, aloe, horse chestnut and other vegetable extracts solutions, urea, uric acid, ammonia, glucosamine, creatine, DNA, RNA, and other nucleic acid related substances.

These substances can be used independently, or in combination of two or more. If two or more types are used in combination, a premix of materials blended beforehand can be used.

The body surface protecting composition of the present invention can be in a variety of forms depending on the objective, including liquid, solid, gel, paste, cream, slurry, emulsion, suspension, mist, liquid crystal, powder, or aerosol. However, the forms are not limited to these examples.

The body surface protecting composition of the present invention can be unneutralized or neutralized using a basic substance, depending on the application and objective.

The multi-chain multiple hydrophilic group-type compound will have enhanced water solubility if neutralized by a basic substance, so when the body surface protecting composition of the present invention is a water based composition, neutralizing is preferable for the sake of handling.

In this case, the pH of the water based composition is preferably adjusted to from 3 to 12 by adjusting the degree of a neutralization using the basic substance, but a pH from 4.5 to 11 is more preferable, and a pH from 5 to 8 is still more preferable.

The body surface protecting composition of the present invention can be used as an agent to protect the surface of the body, or can be broadly used as a composition that contact with humans such as cosmetics and toiletries or medicines.

In the present invention, a body surface protecting agent refers to a substance that is applied externally to the surface of the body (skin or hair) and prevents or reduces damage to the surface of the body.

In the present invention, the term cosmetics and toiletries refers to quasi drug and cosmetic products as defined in the Japanese Pharmaceutical Affairs Law, and specific examples include quasi drug such as mouthwash, underarm deodorant, bath dusting powder, hair growth stimulants, hair removal products, hair dyes, permanent wave solutions, bathing salts, medicinal cosmetics, and medicated toothpaste; and cosmetics, such as cleaning cosmetics such as cosmetic soaps, facial washes (as cream, paste, liquid, gel, granule, outer, or aerosol), shampoo, and rinse; scalp and hair cosmetics such as hair dyes, hair treatments (as a cream, mist, oil, gel, or other form and also including split end coating agents), and hair setting agents (hair grease, setting lotion, curling lotion, pomade, stick, fragrant hair oil, hairspray, hair mist, hair liquid, hair foam, hair gel, and water grease); basic cosmetics, such as general creams, emulsions (cleansing cream, cold cream, vanishing cream, and cream, and the like), shaving cream (after shave cream, shaving cream, and the like), skin lotion (hand lotion, general cosmetic lotion, and the like), cologne, shaving lotion (after shaving lotion,

shaving lotion and the like), cosmetic oils, and packs; makeup cosmetics such as skin whiteners (whitening cream, whitening solids, whitening powder, talcum powder, whitening paste, baby powder, body powder, whitening water, and the like), powder, foundation (as cream, liquid, or solid, or the like), rouge, eyebrow pencil, eye cream, eye shadow, and mascara; perfumes such as standard perfume, paste perfume, and powder perfume; gel, liquid, or ceramic type fragrances, air fresheners, and deodorizers; suntan and sunblock cosmetics such as suntan or sunblock creams, suntan or sunblock lotions, and suntan or sunblock oils; nail cosmetics such as nail creams, fingernail polish, and fingernail polish remover; eyeliner cosmetics; mouth and lip cosmetics such as lipstick and lip creams; oral cosmetics such as toothpaste; and bath cosmetics such as bath salt, bath oil, and bubble bath.

Various types of materials that are normally used in external preparations such as cosmetics and toiletries or medicinal products can be added to the body surface protecting composition of the present invention based on the application and objectives, to the degree that the object of the present invention is not hindered.

Specific examples include dispersing agents such as natural gums like arabia gum and tragacanth gum, glucosides such as saponin, cellulose derivatives such as ) methylcellulose, carboxycellulose, and hydroxymethylcellulose, natural polymers such as lignin solfonate and shellac, anionic polymers such as polyacrylate, styreneacrylic acid copolymer salts, vinyl naphthalene-maleic acid copolymer salts, sodium salts of β-naphthalene sulfonate formalin condensates, and phosphates, and nonionic polymers such as polyvinyl alcohol, polyvinylpyrrolidone, and polyethylene glycol; high level fatty acid salt (soap); high-level fatty acids salts with a hydrophobic group containing between 8 and 20 carbons; N-acylamino acid based anionic surfactants (where the acyl group has between 8 and 20 carbons as described above, and the component amino acid is an amino acid such as the aforementioned acidic amino acids like glutamic acid and aspartic acid, as well as glycine, alanine, valine, leucine, isoleucine, proline, methionine, cystine, tryptophan, tyrosine, phenylalanine, asparagine, glutamine, serine, threonine, oxyproline, β-aminopropionic acid, γ-aminobutanoic acid, anthranilic acid, m-aminobenzoic acid, and p-aminobenzoic acid); anionic surfactants such as alkyl ether carboxylates, amide ether carboxylates, alkyl sulfate ester (AS), polyoxyethylene alkyl ether sulfate esters (AES), alkyl ether sulfate, sulfates of high-level fatty acid esters, sulfates of high-level fatty acid alkylolamides, sulfated oils, polyoxyethylenated phenyl ether sulfates, α-olefin sulfonates (AOS), alkylbenzene sulfonates, alkylnaphthalene sulfonates, alkyl sulfonates (SAS), dialkylsulfosuccinate, α-sulfonated fatty acid salts, alkane sulfonates, sulfonates of higher-level fatty acid esters, α-sulfonated fatty acid salts, sulfonates of higher-level fatty acid amides, N-acyl-N-alkyltaurate, N-acyl-N-methyltaurate, alkyl phosphates, alkyl ether phosphates, polyoxyethylene alkyl ether phosphates, polyoxyethylene alkylphenyl ether phosphates, and naphthalenesulfonate formalin condensate; amphoteric surfactants such as alkylbetaines, alkylamidebetaines, alkylsulfobetaines, imidazolinium betaines, and lecithins; oxygenated ethylene condensate type nonionic surfactants such as polyoxyethylene alkyl ether (AE), polyoxyethylene alkylphenyl ether, polyoxyethylene polystearylphenyl ether, polyoxyethylene polyoxypropylene glycol, polyoxyethylene polyoxypropylene alkyl ether, polyoxyalkylene fatty acid ester, polyoxyalkylene sorbitan fatty acid ester, polyoxyalkylene fatty acid alkanolamide, polyoxyalkylene alkyl glycoside, polyoxyalkylene harden castor oil, polyoxyalkylene alkylamine, and polyoxyalkylene alkylphenyl ether; polyhydric alcohol ester type nonionic surfactants such as polyhydric alcohol fatty acid part ester, polyoxyethylene polyhydric alcohol fatty acid part ester, polyoxyethylene fatty acid ester, (poly)glycerin fatty acid ester, polyoxyethylene hardened castor oil, polyoxyethylene alkylamine, triethanolamine fatty acid part ester, alkyl polyglycoside, propylene glycol fatty acid ester, sorbitan fatty acid ester, and sucrose or the like fatty acid esters; nonionic surfactants such as fatty acid alkanolamide, sugar amine acyl compounds, triethanolamine fatty acid part ester, fatty acid alkylolamide, and alkylamine oxide; cationic surfactants such as primary through tertiary fatty acid amine salts, alkylammonium chloride, tetraalkyl ammonium salt, trialkylbenzyl ammonium salt, alkylpyridinium salt, alkylhydroxylethyl imidazolinium salt, dialkylmorpholinium salt, alkylisoquinolium salt, benzetonium salt, and benzalkonium salt; polymer surfactants such as sodium alginate, starch derivatives, and tragacanth gum; natural surfactants such as phospholipids, lecithin, lanolin, cholesterol, and saponin; oil soluble polymers such as hydroxyethyl cellulose, carboxymethyl cellulose, hydroxyethyl cellulose, hydroxypropyltrimethyl ammonium chloride ether, methylcellulose, ethyl cellulose, hydroxypropyl cellulose, methylhydroxypropyl cellulose, soluble starch, carboxymethyl starch, methyl starch, propylene glycol alginate ester, polyvinyl alcohol, polyvinyl pyrrolidone, polyvinyl methyl either, carboxyvinyl polymer, polyacrylate, guar gum, locust bean gum, queenseed, carrageenan, galactan, arabia gum, pectin, mannan, starch, xanthum gum, dextran, succinoglucane, cardlan, hyaluronic acid, gelatin, casein, albumin, collagen, methoxyethylene maleic anhydride copolymer, amphoteric methacrylate ester copolymer, polydimethylmethylene piperidinium chloride, polyacrylate ester copolymer, polyvinyl acetate, nitrocellulose, and silicone resin; cationic polymers such as cationized cellulose derivative, cationic starch, cationized guar gum derivative, diallylquaternary ammonium salt/acrylamide copolymer, quaternized polyvinyl pyrrolidone derivative, quaternized vinyl pyrrolidone/vinyl imidazole polymer, polyglycol/amine condensate, quaternized collagen polypeptide, polyethyleneimine, cationic silicone polymer, adipic acid/dimethylamino hydroxypropyl diethylene triamine copolymer, polyaminopolyamide, cationic chitin derivative, and quaternized polymer; thickeners and foaming components such as polyethylene glycol fatty acid ester, polyoxyethylene fatty acid ester methylglycoside, and tetradecene sulfonates; sequestering agents such as ethylenediamine tetraacetate and salts thereof, hydroxyethylenediamine triacetate and salts thereof, phosphoric acid, ascorbic acid, succinic acid, gluconic acid, poly phosphates, methaphosphates, and hinokithil (Japanese

cypress extract); preservatives and antimicrobial agents such as paraoxybenzoate esters, benzoic acid and salts thereof, phenoxyethanol, hinokithil (Japanese cypress extract), salicylic acid and salts thereof, sorbic acid and salts thereof, dehydroacetic acid and salts thereof, parachloromethacresol, hexachlorophene, boric acid, resorcin, tribromsalan, orthophenyl phenol, thiram, photosensitizing dye 201, halocarbane, trichlorocarbanide, tocopherol acetate, zinc pyrithione, phenol, isopropylmethylphenol, 2,4,4-trichloro-2-hydroxyphenol, hexachlorophene, chlorohexedine, benzetonium chloride, benzalkonium chloride, cetylpyridinium chloride, decalinium chloride, stearyldimethyl ammonium chloride, stearyltrimethyl ammonium chloride, cetyltrimethyl ammonium chloride, methylbenzetonium chloride, lauryltrimethyl ammonium chloride, lauroyl colamino formylmethyl pyridinium chloride, trichlosan, and biozole; pH adjusting agents such as citric acid, malic acid, adipic acid, glutamic acid, and asparaginic acid; as well as other dandruff and itch preventing agents such as trichlorocarbanilide, salicylic acid, zinc pyrithione, and isopropylmethyl phenol; ultraviolet light absorbers such as benzophenone derivatives, paraminobenzoic acid derivatives, paramethoxycinnamic acid derivatives, and salicylic acid derivatives; whitening agents such as ascorbic acid and salts thereof (alkali metal salts and alkali earth metal salts such as sodium salt, potassium salt, magnesium salts, and calcium salts, as well as ammonium salts and amino acid salts, and the like), ascorbic acid derivatives (alkyl L-ascorbate ester, L-ascorbic acid phosphate ester and salts thereof, L-ascorbic acid-2-sulfate ester and salts thereof, and L-ascorbic acid glucoside and the like), alkoxysalicylic acid and salts thereof (examples of the alkoxy groups include methoxy groups, ethoxy groups, propoxy groups, an isopropoxy groups, butoxy groups, and isobutoxy groups, and the like), hydroquinone glycoside and derivatives thereof (albutin and the like), kojic acid and derivatives thereof, ellagic acid, camomile extract, althaea extract, liver extract, mulberry bark extract, raspberry extract, apple flavonoid, bran extract, vitamin E and derivatives thereof, hinokithiol, placenta extract, leucinol, camomilla ET, glutathione, clove extract, tea extract, astaxanthin, bovine placenta extract, tranexamic acid and derivatives thereof, resorts and derivatives thereof, azulene, and γ-hydroxybutanoic acid; blood circulation enhancers such as swertia extract, cepharanthin, vitamin E and derivatives thereof, and γ-oryzanol; local stimulants such as capsicum tincture, ginger tincture, cantharide tincture, and benzyl nicotinate ester; nutrients such as various vitamins and amino acids; female hormones; hair root activator; antiinflammatories such as glycyrrhetic acid, glycyrrhizinic acid derivative, allantoin, azulene, aminocaproic acid, and hydrocortizone; astringents such as zinc oxide, zinc sulfate, allantoin hydroxy aluminum, aluminum chloride, zinc sulfocarbolate, tannic acid, citric acid, and lactic acid; cooling agents such as menthol and camphor; antihistamines such as diphenhydramine hydrochloride, chlorpheniramine maleate, and glycyrrhizinic acid derivatives; silicone based substances such as polymer silicone and cyclic silicone; antioxidants such as tocopherols, BHA, BHT, gallic acid, and NDGA; sebum inhibitors such as estradiol, estrone, and ethinylestradiol; keratin peelers and dissolvers such as sulfur, salicylic acid, and resorcin; α-hydroxyacids such as glycolic acid, lactic acid, malic acid, tartaric acid, and citric acid; cosmetic colorants such as talc, kaolin, sericite, calcium carbonate, zinc oxide, aluminum oxide, magnesium oxide, zirconium oxide, magnesium carbonate, calcium carbonate, barium carbonate, chromium oxide, chromium hydroxide, tar based colorants, mica, (synthetic) sericite, silicon carbide, boron nitride, titanium dioxide, black titanium oxide, navy blue, red iron oxide, black iron oxide, yellow iron oxide, ultramarine, titanium coated mica, bismuth oxychloride, bengara, body pigments, ultramarine pink, ultramarine violet, chromium hydroxide, mica titanium, chromium oxide, aluminum cobalt oxide, carbon black, silicic anhydride, magnesium silicate, bentonite, (synthetic) mica, zirconium oxide, (meta) magnesium aluminosilicate, calcium aluminosilicate, polyethylene powder, nylon powder, polymethylmethacrylate, polyethylene terephthalate-polymethyl methacrylate layered powder, acrylonitrile-methacrylic acid copolymer powder, vinylidene chloride-methacrylic acid copolymer powder, wool powder, silk powder, crystalline cellulose, N-acyllysine, polymethylsilsesquioxane powder, plant fruit or peel powders, bismuth oxychloride, mica titanium, iron oxide coated mica, iron oxide mica titanium, organic pigment treated mica titanium, aluminum powder, fine powdered titanium oxide, fine powdered zinc oxide, fine powdered titanium oxide coated mica titanium, fine powdered zinc oxide coated mica titanium, barium sulfate coated mica titanium, carmine, β-carotene, chlorophyll, sunset yellow FCF, ponceau SX, eosine YS, tetrabromofluorescein, rhodamine B, quinoline yellow SS, quinoline yellow WS, alizanine cyanine green, quinizarin green, resolvin B, resolvin BCA, parmatone red, helindon pink CN, phthalocyanine, blue, β-apo-8-carotinal, capsanthin, rilopine, bixin, crocin, canthaxanthin, shisonin, lafanine, ninocyanine, carsamine, safrole yellow, rutine, quercitine, cocoa colorant, riboflavin, laccaic acid, carminic acid, kermesic acid, alizanine, shikonin, alkannin, nikino chrome, hemaglobin, curcumin, and betanin; as well as purified water, pyracantha extract, n-methyl-l-serine, whey, nicotinamide, diisopropylamine dichloroacetate, mevalonic acid, γ-aminobutanoic acid (including γ-amino-p-hydroxybutanoic acid), althaea extract, aloe extract, apricot kernel extract, turmeric extract, oolong tea extract, dried seawater, hydrolyzed wheat powder, hydrolyzed, silk, carrot extract, cucumber extract, gentian extract, yeast extract, rice germ extract, comfrey extract, caryophyllaceae extract, rehmannia root extract, lithospermum root extract, white birch extract, peppermint extract, swertia extract, bisabolol, propolis, luffa cylindrica extract, tilia platyphyllos flower extract, hop extract, horse chestnut extract, sapindus mukurossi peel extract, balm mint leaf extract, eucalyptus leaf extract, saxifraga sarementosa extract, rosemary extract, matricaria chamomilla extract, royal jelly extract, seaweed, rice bran, liver, citrus unshiu peel, angelica acutiloba, crushed peach bell, sphingolipids, guaiazulene, and vitamin c and the like.

Of the aforementioned materials, fatty acid diethanaolamide, polyoxyethylene dioleic acid methylglucoside, polyethylene glycol distearic acid, tetradecenesulfonate, myristates, and myristyldimethylamine are useful in cleaning

applications from the viewpoint of increasing the viscosity and foaming action, and the anionic and cationic surfactants are very useful from the viewpoint of further decreasing irritation.

Examples

The present invention will be described below in further detail using examples. Evaluation of the test samples for both the examples of the present invention and the comparative examples was carried out as shown below.

1. Skin Barrier Function Recovery Ratio

The skin barrier recovery ratio is defined as shown below using the transepidermal water loss (TEWL (g/Hr-$m^2$)), and evaluated based on the following criteria. The TEWL was measured using a Cutometer MPA 580 manufactured by COURAGE + KHAZAKA Electronic GMBH Corp..

(Definition of Skin Barrier Function Recovery Ratio)

$$\text{TEWL recovery ratio (\%)}=[((TEWL_{SDS}) - (\text{TEWL over time}))/((TEWL_{SDS}) - (TEWL_0))] \times 100$$

Herein, $TEWL_0$, $TEWL_{SDS}$, and TEWL over time are defined as shown below. $TEWL_0$: The TEWL was measured on the forearm of a panel of 5 healthy males. TE $WL_0$ was defined as the initial average value of TEWL for the panel.

$TEWL_{SDS}$: A 5 mass% concentration of sodium lauryl sulfate (SDS) was applied to the forearm of each panel member in order to induce a condition of reduced skin barrier function and rough dry skin, and then the TEWL value was measured. The average value for the TEWL at this time was defined as $TEWL_{SDS}$. This SDS process was carried out to the extent that the average value of TEWL changed from 7 g/$m^2$hr for the healthy skin to 14 - 17 g/$m^2$hr for rough dry skin.

TEWL over time: 2 μL/$cm^2$ of test sample at an appropriate concentration was openly applied twice a day to the aforementioned rough skin, and the TEWL was measured over time for four days. The average value for the TEWL at this time was defined as TEWL over time.

(Skin Barrier Function Recovery Ratio Evaluation Criteria)

| | |
|---|---|
| Skin barrier function recovery ratio is 60% or higher: | ⊗ |
| Skin barrier function recovery ratio is 40% or higher and less than 60%: | ○ |
| Skin barrier function recovery ratio is 20% or higher and less than 40%: | Δ |
| Skin barrier function recovery ratio is less than 20%: | × |

2. Horny layer Condition

Rough dry skin condition was induced in the same way as the evaluation for the skin barrier function recovery rate on the forearm of 5 healthy males, and then the test sample was openly applied, and the condition of the horny layer was observed four days later using a microscope (trademark, USB Microscope M2-S; manufactured by Scalar Corp.) at a zoom of 50X. The condition of the horny layer that was observed in this manner was evaluated according to the following criteria, and based on the average value of a panel of five persons, and evaluation of ○ was made if the average value was less than 1 point, Δ if the average value was 1 or higher but less than 2, and × if the average value was 2 or higher.

| | |
|---|---|
| Horny layer cells, skin ridges, skin grooves are clearly arranged: | 0 points |
| Horny layer cells, skin ridges, skin grooves are arranged: | 1 points |
| Horny layer cells, skin ridges, skin grooves are somewhat irregular: | 2 points |

(continued)

| | |
|---|---|
| Horny layer cells, skin ridges, skin grooves are visible but irregular: | 3 points |
| Horny layer cells, skin ridges, skin grooves are blurred and irregular: | 4 points |

3. Human Patch Results

0.1 g of test sample was impregnated into wax paper of 1 $cm^2$, and plastered in a covered condition on healthy skin on the forearm of a panel of 5 healthy males for 24 hours. After 24 hours, the TEWL was measured and the results of this human patch test were evaluated by the following criteria. A large increase rate in TEWL means that the sample tends to destroy the barrier function and cause rough dry skin.

○: Average increasing rate of TEWL is below 10%
Δ: Average increasing rate of TEWL is 10% or higher, but less than 30%
x: Average increasing rate of TEWL is 30% or higher

4. Uniformity, Smoothness, and Stylability of Hair

The test sample was thoroughly applied to a bundle of damaged hair that was prepared by the method shown below, which was then blow dried while combing. Evaluation was carried out by a panel of 10 persons from the viewpoint of "uniformity from hair roots to hair tips", "smoothness of the hair", and the "stylability of the hair". The evaluation criteria for "uniformity from hair roots to hair tips", "smoothness of the hair", and the "stylability of the hair" was as shown below.

| | |
|---|---|
| 7 or more persons gave an assessment of "good": | ○ |
| From 4 to 6 persons gave an assessment of "good": | Δ |
| 3 or less persons gave an assessment of "good": | × |

(Method for Preparing Damaged Hair)

A bundle of damaged hair was prepared by repeating 4 cycles of a process consisting of applying a permanent wave treatment, brushing 100 times, bleaching, brushing 100 times,to a bundle of healthy hair (15 cm, 1 g).

5. Hair Damage Recovery Ratio

The hair damage recovery rate was defined by the following formula using damage recovered hair prepared by immersion in a test sample for 1 minute and then drying the aforementioned damaged hair.

$$\text{Hair damage recovery ratio (\%)} = \text{tensile strength of damage recovered hair/tensile strength of healthy hair} \times 100$$

Wherein the tensile strength is defined as the breaking strength per unit cross-section area, and the breaking strength and the cross-section area of the hair are measured as shown below.

(a) Breaking Strength

The breaking strength was determined using a tensilon tensile tester.

- Tensile speed - - -20 mm/min
- Test hair length - - -40 mm
- Chart speed- - - 100 mm/min
- N number = 5

(b) Cross-sectional area of a hair

After having measured the breaking strength, the hair was cut at a position approximately 20 mm from the tip

using a razor, and the area was determined by measuring a microscope image.

6. Liquid Crystal Condition

A test sample was dispersed by heating, and then observed at a magnification of 40X using a cross nicol polarized light microscope (CX31-P manufactured by Olympus Corporation), and the liquid crystal condition was evaluated according to the following criteria.

| | |
|---|---|
| 30 or more maltese cross images can be observed from all view | ⊗ |
| Maltese cross images can be observed from all view, but ⊗ conditions not met | ○ |
| Maltese cross image can be observed depends on view | Δ |
| Maltese cross image cannot be observed from any view | × |

[Acyl Compound Manufacturing 1]

9.1 g of L-lysine monohydrochloride (0.05 mol) was blended with 57 g of water. This solution was adjusted to a pH of 10 - 11 with a 25% aqueous solution of sodium hydroxide, while maintaining the reaction temperature at 5°C, and a reaction was carried out by adding 31.1 g (0.1 mol) of N-lauroyl-L-glutamic anhydride over 2 hours while stirring. After continuous stirring of the solution for 2 hours, tertiary butanol was added until the concentration in the solution was 20 mass%, and then 75% sulfuric acid was added in drops to adjust the pH to 2, and the temperature of the solution was adjusted to 65°C. After the addition of the sulfuric acid was complete, stirring was stopped, the solution was allowed to sit for 20 minutes at 65°C, and then the solution separated into an organic layer and an aqueous, layer, and the organic layer was separated. Tertiary butanol and water were added to the separated organic layer, and the temperature was maintained at 65°C while stirring for 20 minutes. After stopping stirring, the solution was allowed to sit and separate into an organic layer and an aqueous layer. After repeating the same water washing operation on the organic layer obtained, the solvent was removed from the organic layer, and then the solution was neutralized to a pH of 6.7 (25°C) using sodium hydroxide followed by adjusting to a solid content or 30 mass%, and the solution was dried to obtain the compounds (mixtures) expressed in the following formula (5).

[C 20]

....(5)

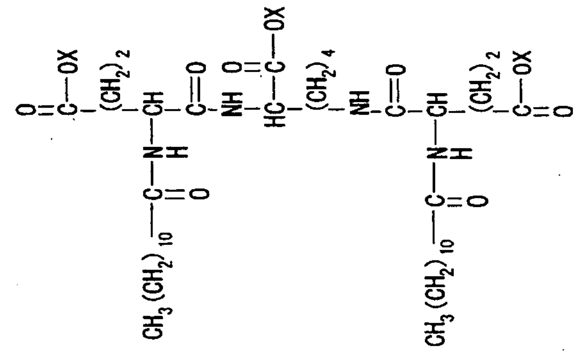

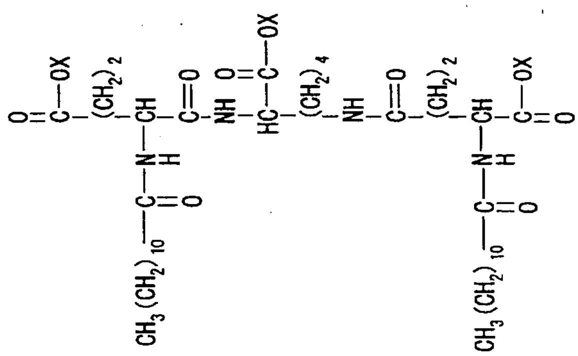

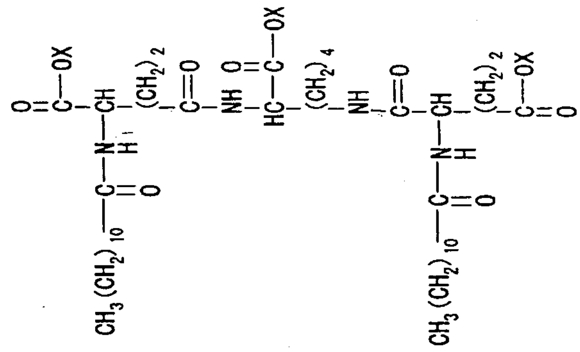

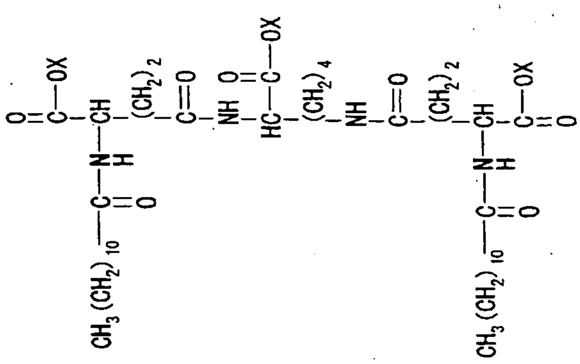

(In formula (5), X independently represents either H or Na.)

[Acyl Compound Manufacturing 2]

The compounds (mixture) as expressed in the following formula (6) were obtained using the same method and

conditions as example 1, with the exception that 31.1 g of N-lauroyl-L-glutamic anhydride was replaced with 39.5 g (0.1 mol) of N-stearoyl-L-glutamic anhydride, and instead of neutralizing the pH of the solution to 6.7 (25°C) and adjusting the solid content to 30 mass%, the pH was neutralized to 7 (60°C), and the solid content was adjusted to 20 mass%.

[C 21]

...(6)

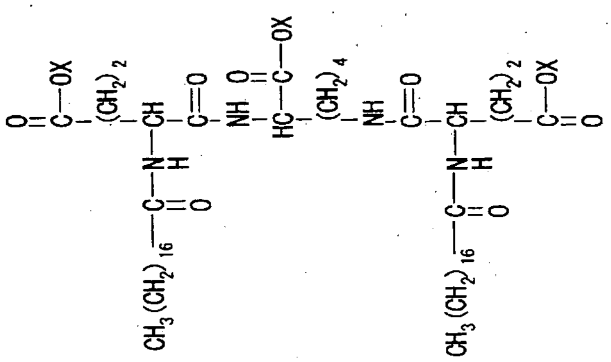

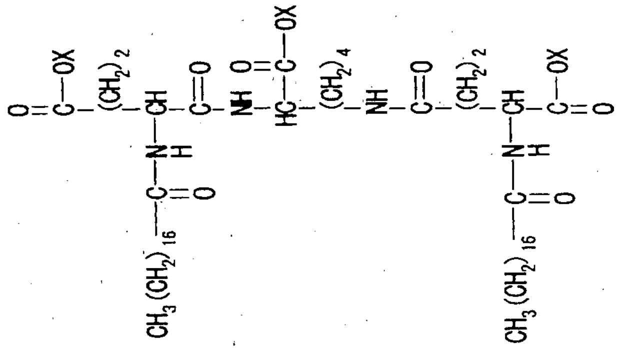

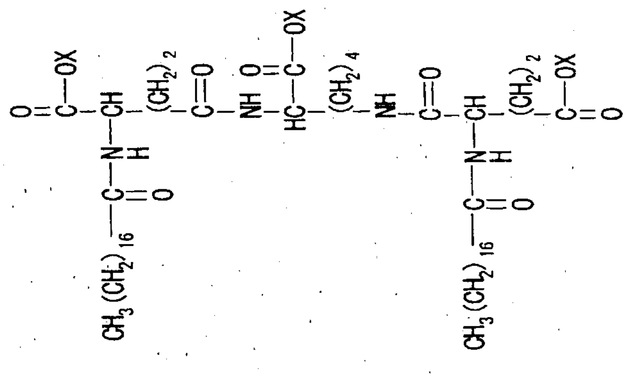

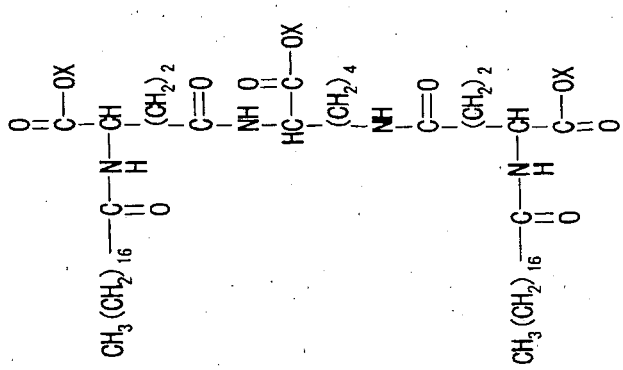

(In formula (6), X independently represents either H or Na.)

[Acyl Compound Manufacturing 3]

The compounds (mixture) as shown in the following formula (7) were obtained using the same method and conditions as example 1, with the exception that the 31.1 g of N-lauroyl-L-glutamic anhydride was replaced with 31.1 g (0.1 mol) of N-cocooyl-L-glutamic anhydride, and potassium hydroxide was used for neutralization when the solution was neutralized and adjusted to a solid content of 30 mass% and a pH of 6.7 (25°C).

[C 22]

...(7)

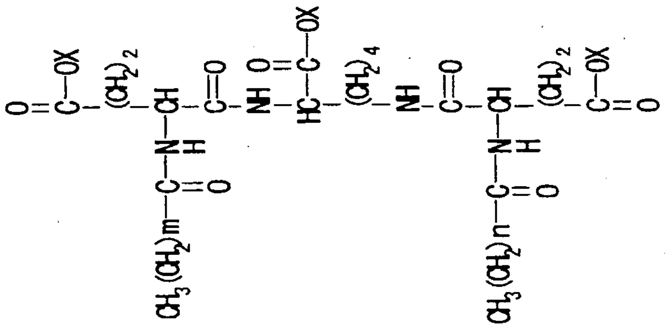

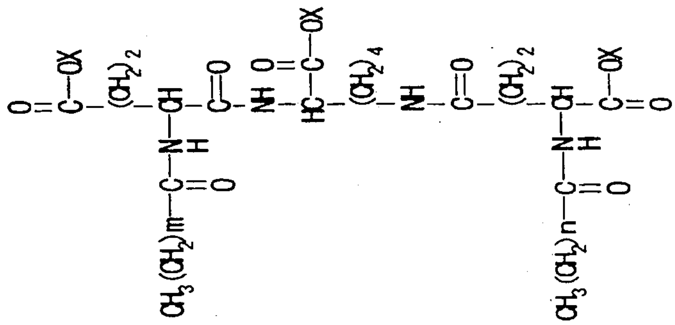

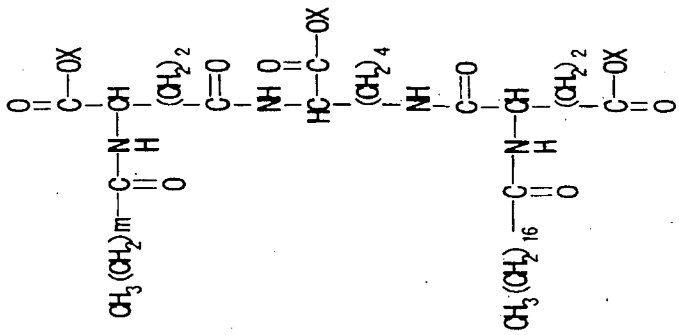

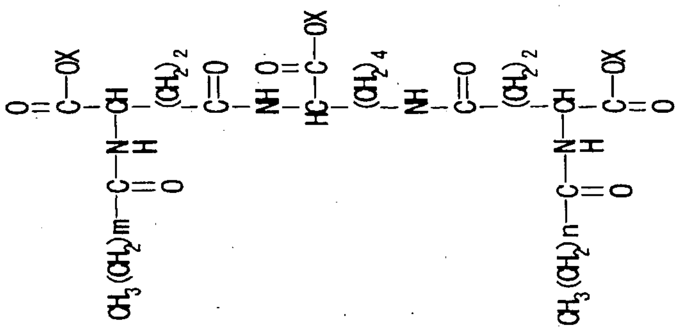

(In formula (7), X independently represents either H or Na, and m and n each independently represent integers from 6 to 16.)

[Examples 1 through 9 and Comparative Examples 1 through 6]

The components shown in Table 1 were combined in the amounts (mass parts) shown in Table 1 to prepare

body surface protecting compositions for examples 1 through 9 and comparative examples 1 through 6. (Total amount was 100 parts, remainder was purified water). The skin barrier function recovery ratio and the condition of each layer was evaluated for each body surface protecting composition. The results are shown in Table 1.

Examples 3 and 4 are according to the invention. The remaining Examples 1, 2 and 5 to 9 are for reference.

[Table 1]

| Formulation | Ex 1* | Ex 2* | Exe 3 | Ex 4 | Ex 5* | Ex 6* | Ex 7* | Ex 8* | Ex 9* | Comp Ex 1 | Comp Ex 2 | Comp Ex 3 | Comp Ex 4 | Com Ex 5 | Comp Ex 6 |
|---|---|---|---|---|---|---|---|---|---|---|---|---|---|---|---|
| Compound of manufacturing 1 | 5 | -- | -- | 5 | 1 | -- | -- | 1 | 2.5 | -- | -- | -- | -- | -- | -- |
| Compound of manufacturing 2 | -- | 5 | -- | -- | -- | 1 | -- | -- | -- | -- | -- | -- | -- | -- | -- |
| Compound of manufacturing 3 | -- | -- | 5 | -- | -- | -- | 1 | -- | -- | -- | -- | -- | -- | -- | -- |
| Sodium hyaluronate | -- | -- | -- | -- | -- | -- | -- | 2.5 | -- | -- | -- | 5 | -- | -- | -- |
| Glycerin | -- | -- | -- | -- | -- | -- | -- | -- | 2.5 | 5 | -- | -- | -- | -- | -- |
| Cholesterol | -- | -- | -- | 0.5 | 0.5 | -- | -- | -- | -- | -- | -- | -- | -- | -- | -- |
| Urea | -- | -- | -- | -- | -- | -- | -- | -- | -- | -- | 5 | -- | -- | 4 | -- |
| Sodium N-lauroyl-L-glutamate | -- | -- | -- | -- | -- | -- | -- | -- | -- | -- | -- | -- | 5 | -- | -- |
| L-lysine | -- | -- | -- | -- | -- | -- | -- | -- | -- | -- | -- | -- | -- | 1 | -- |
| Purified water | Remainder | Remainder | Remainder | Remainder | Remainder | Remainder | Remainder | Remainder | Remainder | Remainder | Remainder | Remainder | Remainder | Remainder | Remainder |
| Barrier recovery ratio (%) | | | | | | | | | | | | | | | |
| After 1 day | ○ | ○ | ○ | ○ | ○ | ○ | ○ | ○ | ○ | × | × | × | × | × | × |
| After 2 days | ⊗ | ⊗ | ⊗ | ⊗ | ○ | ○ | ○ | ○ | ○ | × | × | Δ | Δ | Δ | Δ |
| After 3 days | ⊗ | ⊗ | ⊗ | ⊗ | ⊗ | ○ | ○ | ⊗ | ○ | Δ | Δ | Δ | Δ | Δ | Δ |
| After 4 days | ⊗ | ⊗ | ⊗ | ⊗ | ⊗ | ⊗ | ⊗ | ⊗ | ⊗ | Δ | Δ | Δ | X | X | Δ |

(continued)

| Formulation | Ex 1* | Ex 2* | Exe 3 | Ex 4 | Ex 5* | Ex 6* | Ex 7* | Ex 8* | Ex 9* | Comp Ex 1 | Comp Ex 2 | Comp Ex 3 | Comp Ex 4 | Com Ex 5 | Comp Ex 6 |
|---|---|---|---|---|---|---|---|---|---|---|---|---|---|---|---|
| Condition of horny layer (after 4 days) | ⊗ | ⊗ | ⊗ | ○ | ○ | ○ | ○ | ○ | ○ | Δ | × | Δ | × | × | × |
| * reference examples | | | | | | | | | | | | | | | |

[Examples 10 through 12 (for reference) and Comparative Examples 7 through 9]

Body surface protecting composition were prepared by combining the components shown in Table 2 in the amount (mass part) shown in Table 2. A human patch test was performed and the effect on the barrier function was confirmed for each composition for protecting human body surface. The results are shown in Table 2.

[Table 2]

| Formulation | Example 10** | Example 11** | Example 12** | comparative Example 7 | Comparative Example 8 | Comparative Example 9 |
|---|---|---|---|---|---|---|
| Compound of manufacturing 1 | 5 | -- | -- | -- | -- | -- |
| Compound of manufacturing 2 | -- | 5 | -- | -- | -- | -- |
| Compound of manufacturing 3 | -- | -- | 5 | -- | -- | -- |
| Sodium lauroyl glutamate | -- | -- | -- | 5 | -- | 4 |
| Potassium cocoyl glycine | -- | -- | -- | -- | 5 | -- |
| L-lysine | -- | -- | -- | -- | -- | 1 |
| Purified water | Remainder | Remainder | Remainder | Remainder | Remainder | Remainder |
| Human patch test | | | | | | |
| After 1 day | ○ | ○ | ○ | Δ | × | Δ |
| After 2 days | ○ | ○ | ○ | × | × | × |
| * The values in the table are shown as mass%. The total was made to be 100 mass%.<br>** reference examples | | | | | | |

Compositions containing the multi-chain multiple hydrophilic group-type compound of manufacturing examples 1 through 3 were confirmed as not having a negative effect on the barrier function. On the other hand, a composition containing sodium lauroyl glutamate, a composition containing sodium lauroyl glutamate and lysine, and a composition containing potassium cocoyl glycine, which are single chain-type compounds that have basic constitutional units similar to the multi-chain multiple hydrophilic group-type compounds of examples 1 and 3 were confirmed as degrading the barrier function.

From the above, it was confirmed that multi-chain multiple hydrophilic group-type compounds have an effect on improving the barrier function of skin and hair originating from their overall structure.

[Example 13] (reference example)

A cream was prepared as shown below. Stability, skin irritability, and effect of improving the horny layer condition of the cream were all favorable.

| (Component) (mass parts) | |
|---|---|
| (1) Stearic acid | 6 |
| (2) Cetyl alcohol | 4 |
| (3) Butyl stearate | 6 |
| (4) Propylene glycol | 5 |
| (5) Glycerin monostearate | 2 |

(continued)

| (Component) | (mass parts) |
|---|---|
| (6) potassium hydroxide | 0.3 |
| (7) Acyl compound of manufacturing | 11 |
| (8) Perfume as appropriate | |
| (10) Preservative and antioxidant | as appropriate |
| (11) Purified water | 75.7 |

[Example 14] (reference example)

A cream was prepared as shown below. Stability, skin irritability, and effect of improving the horny layer condition of the cream were all favorable.

| (Component) | (mass parts) |
|---|---|
| (1) Stearic acid | 2 |
| (2) Stearyl alcohol | 5 |
| (3) Hydrated lanolin | 4 |
| (4) Squalane | 10 |
| (5) Octyldodecanol | 9 |
| (6) 1,3-butylene glycol | 6 |
| (7) polyethylene glycol (mw 1500) | 5 |
| (8) Acyl compound of manufacturing 2 | 0.5 |
| (9) Perfume as appropriate | |
| (10) Preservative and antioxidant | as appropriate |
| (11) Purified water | 53 |

[Example 15] (reference example)

A lotion was prepared as shown below. Stability, skin irritability, and effect of improving the horny layer condition of the lotion were all favorable.

| (Component) | (mass parts) |
|---|---|
| (1) Stearic acid | 2 |
| (2) Cetyl alcohol | 1.5 |
| (3) Vaseline | 4 |
| (4) Squalane | 4 |
| (5) Glycerol tri-2-ethylhexanoate ester | 3 |
| (6) Dipropylene glycol | 5 |
| (7) polyethylene glycol (mw 1500) | 3 |
| (8) Acyl compound of manufacturing 3 | 1 |
| (9) Triethanolamine | 1 |
| (10) Perfume as appropriate | |
| (11) Preservative and antioxidant | as appropriate |
| (12) Purified water | 75.5 |

[Example 16] (reference example)

An emulsion was prepared as shown below. Stability, skin irritability, and effect of improving the horny layer condition of the emulsion were all favorable.

| (Component) | (mass parts) |
|---|---|
| (1) Cetyl alcohol | 1 |
| (2) Beeswax | 0.5 |

(continued)

| (Component) (mass parts) | |
|---|---|
| (3) Vaseline | 2 |
| (4) Squalane | 5 |
| (5) Dimethyl polysiloxane | 3 |
| (6) Ethanol | 5 |
| (7) Glycerin | 5 |
| (8) Acyl compound of manufacturing 1 | 1 |
| (9) 1,3-butylene glycol | 3 |
| (10) Carboxyvinyl polymer | 0.2 |
| (11) Perfume as appropriate | 0.2 |
| (12) Preservative and antioxidant | as appropriate |
| (13) Purified water | 74.3 |

[Example 17] (reference example)

A gel was prepared as shown below. Stability, skin irritability, and effect of improving the horny layer condition of the gel were all favorable.

| (Component) (mass parts) | |
|---|---|
| (1) polyethylene glycol (mw 1500) | 9 |
| (2) Dipropylene glycol | 7 |
| (3) Methyl cellulose | 0.2 |
| (4) Carboxyvinyl polymer | 0.4 |
| (5) Acyl compound of manufacturing | 31 |
| (6) potassium hydroxide | 0.1 |
| (7) Perfume as appropriate | |
| (8) Preservative and antioxidant | as appropriate |
| (9) Chelating agent | as appropriate |
| (10) Purified water | 82.3 |

[Example 18] (reference example)

A facial mask was prepared as shown below. Stability, skin irritability, and effect of improving the horny layer condition of the facial mask were all favorable. (Component) (mass parts)

| (Component) | (mass parts) |
|---|---|
| (1) 1,3-butylene glycol | 5 |
| (2) Polyvinyl alcohol | 15 |
| (3) Carboxymethyl cellulose | 5 |
| (4) Ethanol | 12 |
| (5) Acyl compound of manufacturing 1 | 1 |
| (6) Preservative | as appropriate |
| (7) Perfume | as appropriate |
| (8) Purified water | amount to make the total 100 parts |

[Example 19] (reference example)

A lotion was prepared as shown below. Stability, skin irritability, and effect of improving the horny layer condition of the lotion were all favorable.

| (Component) | (mass parts) |
|---|---|
| (1) Ethanol | 55 |
| (2) Dipropylene glycol | 2.5 |
| (3) Acyl compound of manufacturing 2 | 1 |
| (4) Antimicrobial agent | as appropriate |
| (5) Perfume | as appropriate |
| (6) Purified water | to make 100 parts |

[Examples 20 through 24, Comparative Examples 10, 11] 8Examples 20 and 23 are reference examples)

(Preparation of Body surface protecting composition for Example 20 and Comparative Example 10)

The body surface protecting composition for example 20 was prepared by combining the components in the amount (mass part) shown in Table 3. Comparative Example 10 contained only purified water.[0129] (Preparation of Body surface protecting composition for Examples 21 through 23 and Comparative Example 11)

The body surface protecting compositions for Example 21 through 23 were prepared by combining the components shown in Table 3 and the following Base Emulsion A in the amount (mass part) shown in Table 3. Base Emulsion A was used alone as the Comparative Example 11.

(Base Emulsion A)

| | |
|---|---|
| Liquid paraffin | 10 mass parts |
| SEF-320A (product of Nihon Emulsion Co. Ltd.) | 3 mass parts |
| 1,3-butylene glycol | 5 mass parts |
| Cetyl alcohol | 2 mass parts |
| Water | remainder |

(Preparation of Body surface protecting composition for Example 24)

The body surface protecting composition for Example 24 was prepared by combining the components shown in Table 3 and the following Base Emulsion B in the amount (mass part) shown in Table 3.

(Base Emulsion B)

| | |
|---|---|
| Squalane | 10 mass parts |
| 1,3-butylene glycol | 5 mass parts |
| Cetyl alcohol | 2 mass parts |
| Water | remainder |

(Evaluation)

The skin barrier function recovery ratio, hair uniformity, smoothness, and stylability, and liquid crystal condition were evaluated for Examples 20 through 24 and Comparative Examples 10 and 11. The results are shown in Table 3.

[Table 3]

| Formulation | Example 20** | Example 21 | Example 22 | Example 23** | Example 24 | Comparative Example 10 | Comparative Example 11 |
|---|---|---|---|---|---|---|---|
| Compound of manufacturing 1 | 0.5 | -- | 0.5 | 0.5 | 1 | -- | -- |
| Compound of manufacturing 2 | -- | 0.5 | -- | -- | -- | -- | -- |
| Lauric acid | -- | -- | 0.2 | -- | 0.3 | -- | -- |
| Cholesterol | -- | 0.3 | 0.3 | -- | 0.5 | -- | -- |
| Purified water | Remainder | -- | -- | -- | -- | Remainder | -- |
| Base Emulsion | -- | Remainder | Remainder | Remainder | Remainder | -- | Remainder |
| Barrier recovery ratio (%) | | | | | | | |
| After 3 days | × | Δ | ○ | × | ○ | × | × |
| After 7 days | Δ | ⊗ | ⊗ | ○ | ⊗ | × | × |
| After 10 days | ○ | ⊗ | ⊗ | ⊗ | ⊗ | Δ | ○ |
| After 14 days | ⊗ | ⊗ | ⊗ | ⊗ | ⊗ | ○ | ○ |
| Uniformity of hair | ○ | ○ | ○ | ○ | ○ | × | × |
| Smoothness of hair | ○ | ○ | ○ | ○ | ○ | Δ | Δ |
| Stylability of hair | ○ | ○ | ○ | ○ | ○ | Δ | Δ |
| Liquid crystal condition observation results | ○ | ⊗ | ⊗ | -- | ⊗ | × | × |
| Overall evaluation | ○ | ⊗ | ⊗ | ⊗ to ○ | ⊗ | × | × |

* The values in the table are shown as mass%. The total was made to be 100 mass%.
** reference examples

Formation of liquid crystal was confirmed in the compositions of Examples 20 through 22 and 24, and in particular, formation of spherical crystals were clearly confirmed in the compositions of Examples 21, 22, and 24.

From the above, it was confirmed that a body surface protecting composition containing multi-chain multiple hydrophilic group-type compound (A) of the present invention formed liquid crystals, and it was also confirmed that in the present invention the formation of liquid crystal was facilitated since the composition also contained oil-based component (B). Furthermore, when liquid crystals were formed, in addition to the function of accelerating skin barrier recovery, the effect of improving hair, particularly hair tips, that had been damaged was also confirmed.

Example 25

Body surface protecting compositions were prepared by combining the components shown in Table 4 in the amount (mass part) shown in Table 4. The liquid crystal condition of these compositions was evaluated. The results are shown in Table 4.

[Table 4]

| Compound of manufacturing 1 | Cholesterol | Lauric acid | Base Emulsion (A) | Liquid crystal formation |
|---|---|---|---|---|
| 0.5 | 16 | -- | Remainder | × |
| 0.5 | 12 | -- | Remainder | Δ |
| ** 0.5 | 4.6 | -- | Remainder | Δ |
| ** 0.5 | 2.3 | -- | Remainder | ○ |
| ** 0.5 | 1.7 | -- | Remainder | ○ |
| ** 0.5 | 1.2 | -- | Remainder | ⊗ |
| ** 0.5 | 0.23 | -- | Remainder | ⊗ |
| 0.5 | 0.046 | -- | Remainder | ⊗ |
| 0.5 | 0.031 | -- | Remainder | ○ |
| 0.5 | 0.023 | -- | Remainder | ○ |
| 0.5 | 0.012 | -- | Remainder | Δ |
| 0.5 | 0.005 | -- | Remainder | Δ |
| 0.5 | 0.003 | -- | Remainder | × |
| 0.5 | -- | 8.4 | Remainder | × |
| 0.5 | -- | 6 | Remainder | Δ |
| 0.5 | -- | 2.4 | Remainder | Δ |
| 0.5 | -- | 1.2 | Remainder | ○ |
| 0.5 | -- | 0.90 | Remainder | ○ |
| 0.5 | -- | 0.60 | Remainder | ⊗ |
| 0.5 | -- | 0.12 | Remainder | ⊗ |
| 0.5 | -- | 0.024 | Remainder | ⊗ |
| 0.5 | -- | 0.016 | Remainder | ○ |
| 0.5 | -- | 0.012 | Remainder | ○ |
| 0.5 | -- | 0.0060 | Remainder | Δ |
| 0.5 | -- | 0.0024 | Remainder | Δ |
| 0.5 | -- | 0.0017 | Remainder | × |

* The figures in the table are shown in mass%. The total was made to be 100 mass%.
** according to the present invention

Observations were made at 40X zoom using a cross nicol polarized light microscope.

⊗: 30 or more maltese cross images can be observed from all view
○: Maltese cross images can be observed from all view
Δ: Maltese cross image can be observed depends on view
×: Maltese cross image can not be observed from any view

Formation of liquid crystals was confirmed in the composition where cholesterol (molecular weight 387) and lauric acid (molecular weight 200) were added with a molar ratio to a multi-chain multiple hydrophilic group-type compound (molecular weight 835) of from 1/50 to 50/1, and in particular, formation was clearly confirmed in the composition with a molar ratio of from 1/10 to 10/1, and very remarkable formation of liquid crystals was confirmed in the composition with a molar ratio of from 1/5 to 5/1.

From the above, it was confirmed that a composition containing multi-chain multiple hydrophilic group-type compound (A) of the present invention could easily form a liquid crystal composition if oil-based component (B) was used in combination in a specific range within the ratio.

[Examples 26 through 28, Comparative Examples 12]

Body surface protecting compositions were prepared by combining the components shown in Table 5 in the amount (mass part) shown in Table 5. Comparative Example 12 was purified water alone. The hair damage recovery rate was measured for each composition. The results are shown in Table 5.

[Table 5]

| Formulation | Example 26** | Example 27 | Example 28** | Comparative Example 12 | Untreated damaged hair |
|---|---|---|---|---|---|
| Compound of manufacturing 1 | 0.01 | 0.01 | 0.01 | -- | -- |
| Lauric Acid | -- | -- | 0.0008 | -- | -- |
| Cholesterol | -- | 0.0015 | -- | -- | -- |
| Purified water | Remainder | Remainder | Remainder | Remainder | -- |
| Damage recovery ratio | 94% | 100% | 99% | 83% | 81% |
| * The figures in the table are shown in mass%. The total was made to be 100 mass%.<br>** reference examples | | | | | |

From the above, it was confirmed that a composition containing a multi-chain multiple hydrophilic group-type compound (A) according to the present invention will have an excellent effect of improving hair that has been damaged, if the oil-based component (B) is used in combination in a specific molar rati with respect to compound (A).

INDUSTRIAL APPLICABILITY

The body surface protecting composition of the present invention will accelerate recovery of the barrier function of skin and hair when that function has deteriorated due to skin trouble or the like and will prevent degradation of the barrier function, and therefore can be used by adding to cosmetic compositions, pharmaceutical compositions, and body surface protecting agents.

## Claims

1. A body surface protecting composition, comprising, (A) at least one type of compound that is a multi-chain multiple hydrophilic group-type compound selected from compounds a to 1 below:

compound a

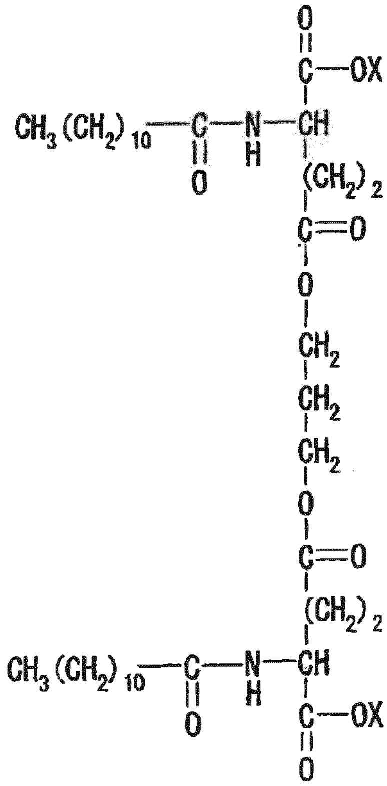

compound b

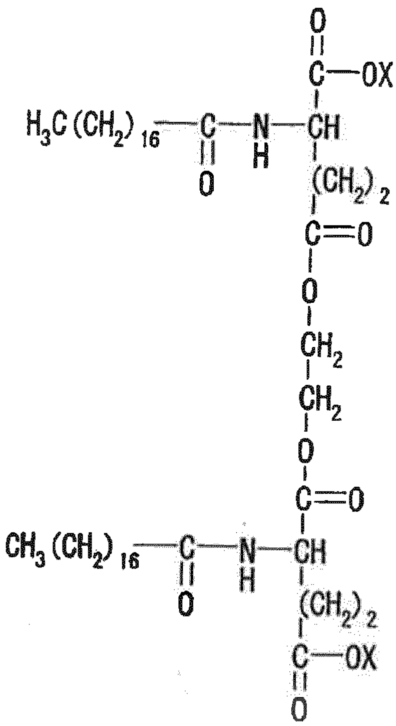

compound C

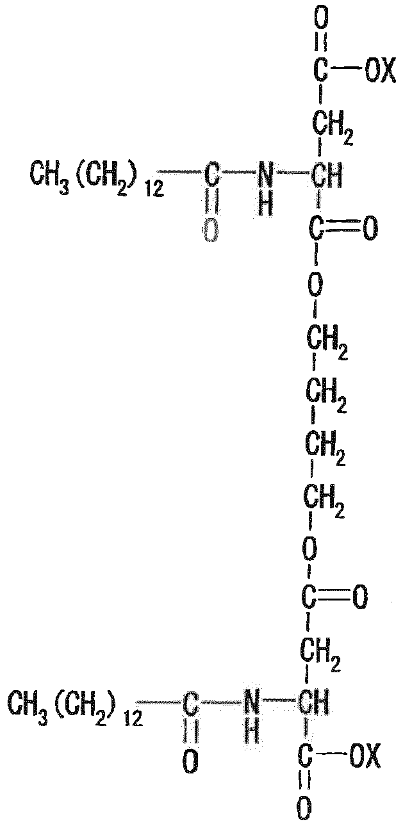

compound **d**

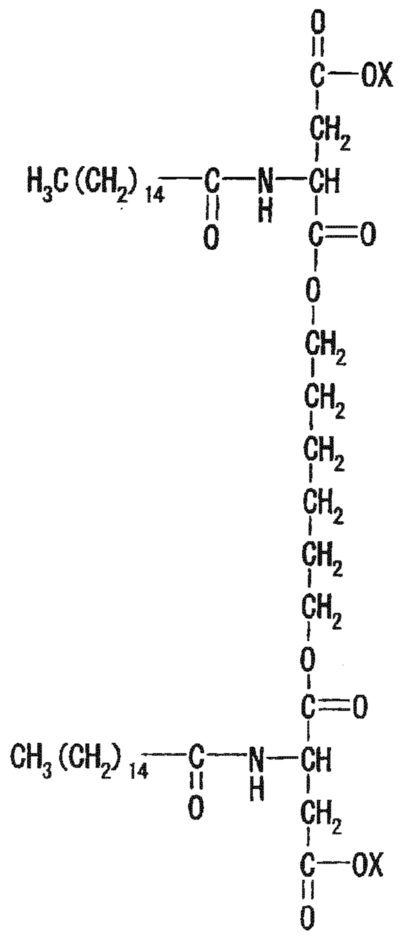

compound e

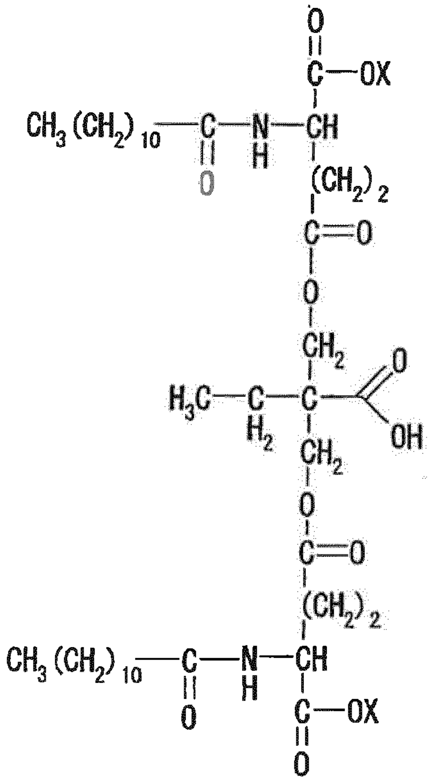

compound f

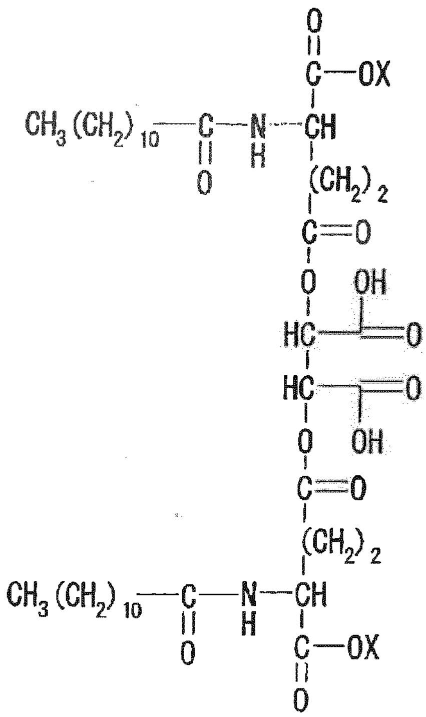

compound g

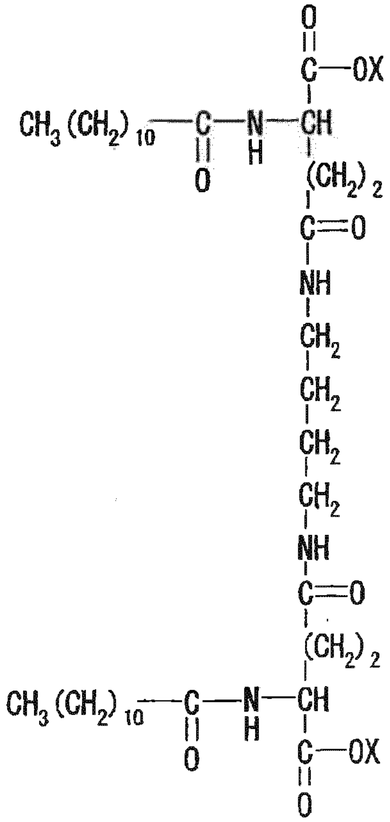

compound h

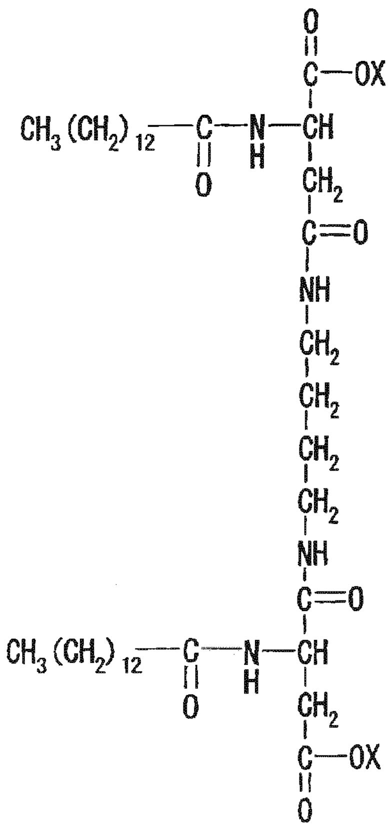

compound i

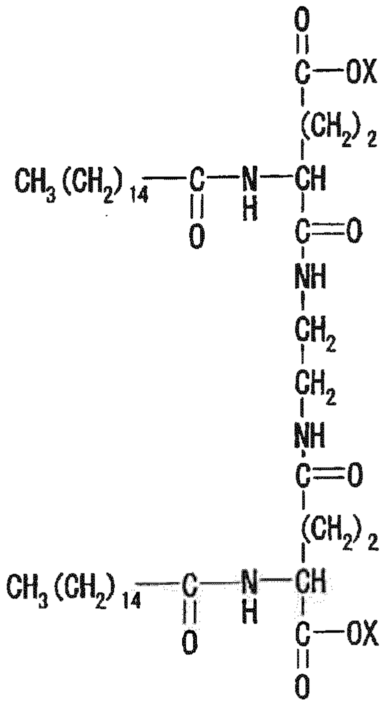

compound j

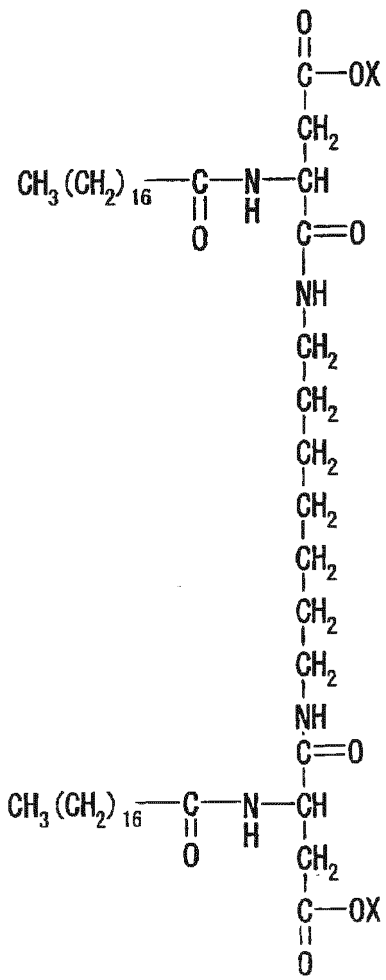

compound k

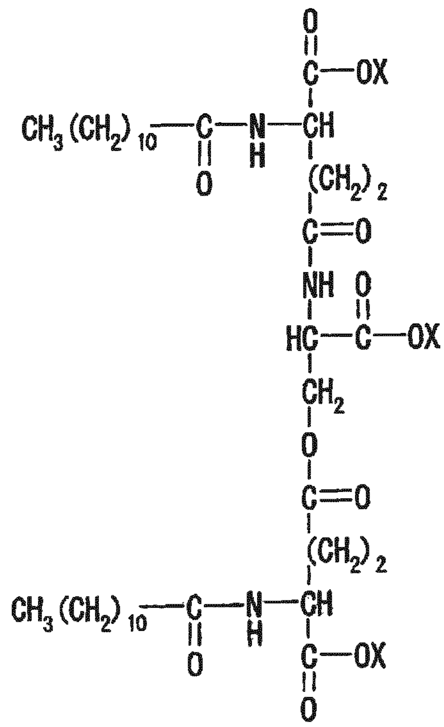

compound l

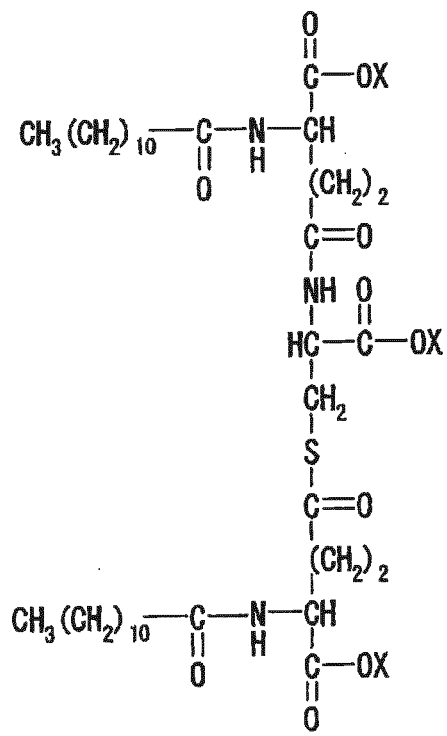

and compounds (mixtures) represented by the following formulae (5) to (7):

....(5)

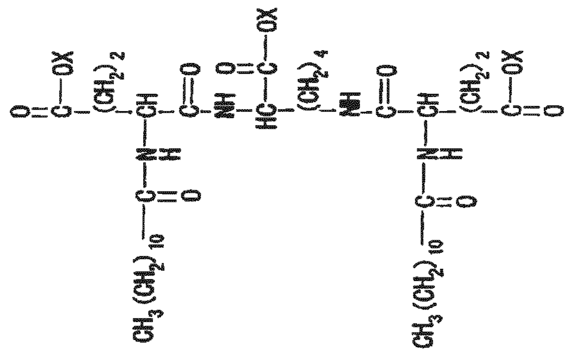

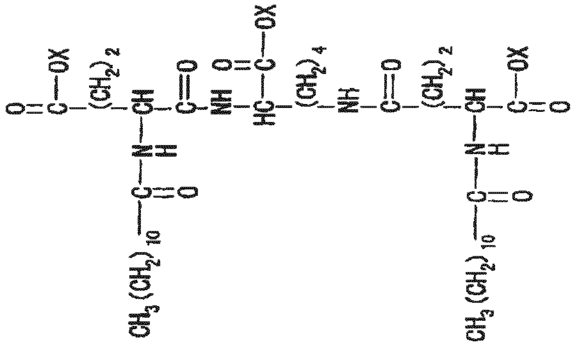

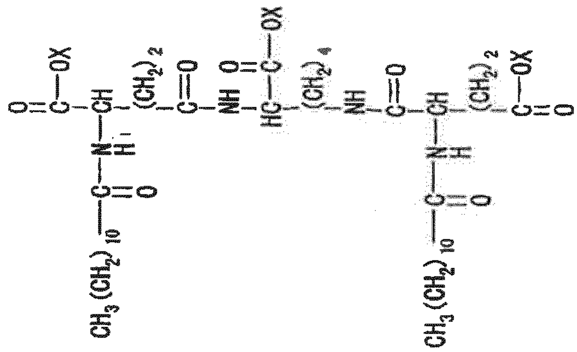

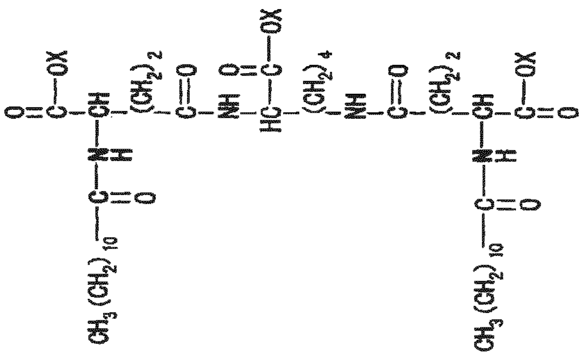

wherein X independently represents either H or Na,

···(6)

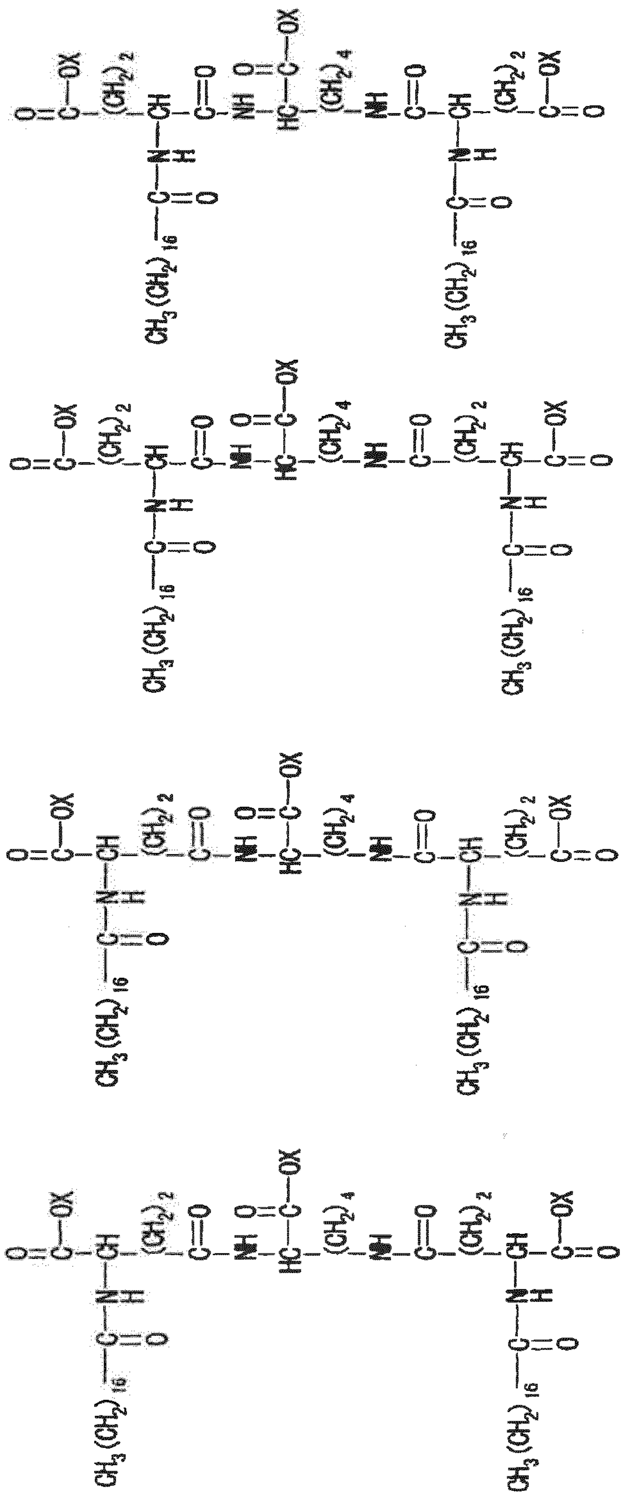

wherein X independently represents either H or Na,

•••(7)

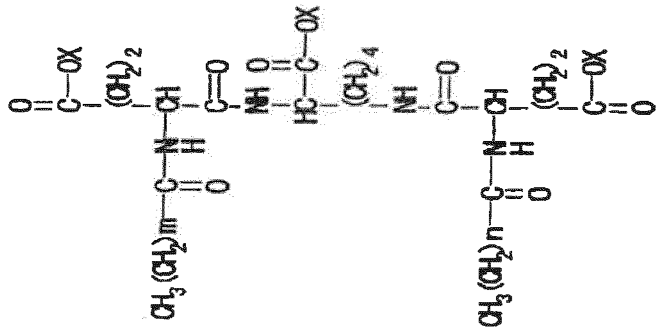

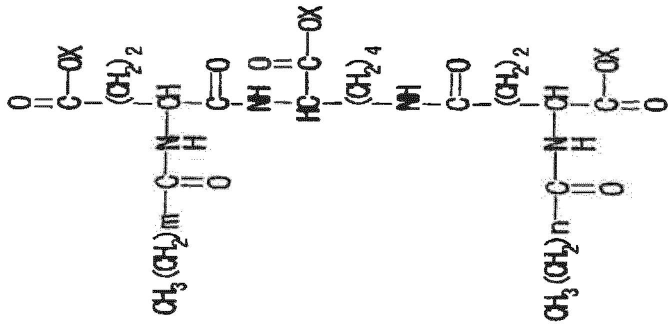

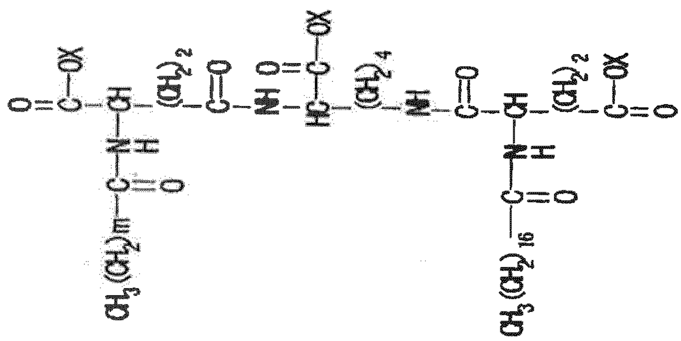

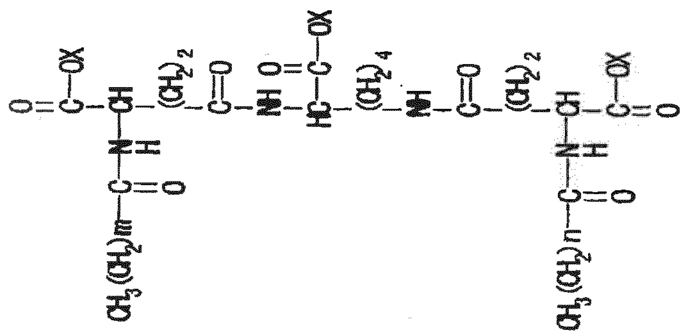

wherein X independently represents either H or Na, and m and n each independently represent integers from 6 to 16 and (B) at least one type of oil-based component, wherein said oil-based component is a liquid crystal forming aid which is at least one type of sterol compound, wherein the molar ratio of said liquid crystal forming aid to (A) (amount of liquid crystal forming aid: amount of (A)) is 1:10 or higher and 10:1 or lower.

**2.** The composition according to Claim 1, wherein said (A) forms liquid crystals.

**3.** The body surface protecting composition according to Claim 1, wherein said sterol compound is at least one selected from a group consisting of cholesterol, cytosterol, lanosterol, phytosterol, dihydrocholesterol, dihydrolanosterol, and dehydrocholesterol and esters thereof.

**4.** The body surface protecting composition according to Claim 3, wherein the molar ratio of said liquid crystal forming aid to (A) (amount of liquid crystal forming aid: amount of (A)) is 1:5 or higher and 5:1 or lower.

**5.** The body surface protecting composition according to any one of Claims 1 through 4, further comprising (C) a horny layer intercellular component and/or (D) a water soluble moisturizing agent.

**6.** An external preparation for reducing or preventing allergenic or inflammatory skin disease, comprising the body surface protecting composition according to any one of Claims 1 through 5.

**7.** A cosmetic toiletry product comprising the body surface protecting composition according to any one of Claims 1 through 5.

**8.** A pharmaceutical product comprising the body surface protecting composition according to any one of Claims 1 through 5.

**9.** A body surface protecting agent comprising the body surface protecting composition according to any one of Claims 1 through 5.

**10.** A use of the body surface protecting composition according to any one of Claims 1 through 5 for manufacturing an external preparation for reducing or preventing allergenic or inflammatory skin disease.

**11.** A use of the body surface protecting composition according to any one of Claims 1 through 5 for manufacturing a cosmetic toiletry product.

**12.** A use of the body surface protecting composition according to any one of Claims 1 through 5 for manufacturing a pharmaceutical product.

**13.** A use of the body surface protecting composition according to any one of Claims 1 through 5 for manufacturing a body surface protecting agent.

## Patentansprüche

**1.** Körperoberflächenschutzzusammensetzung, die (A) mindestens eine Art einer Verbindung, die eine Verbindung mit mehreren Ketten und mehreren hydrophilen Gruppen ist, ausgewählt unter den nachstehenden Verbindungen a bis 1:

Verbindung a

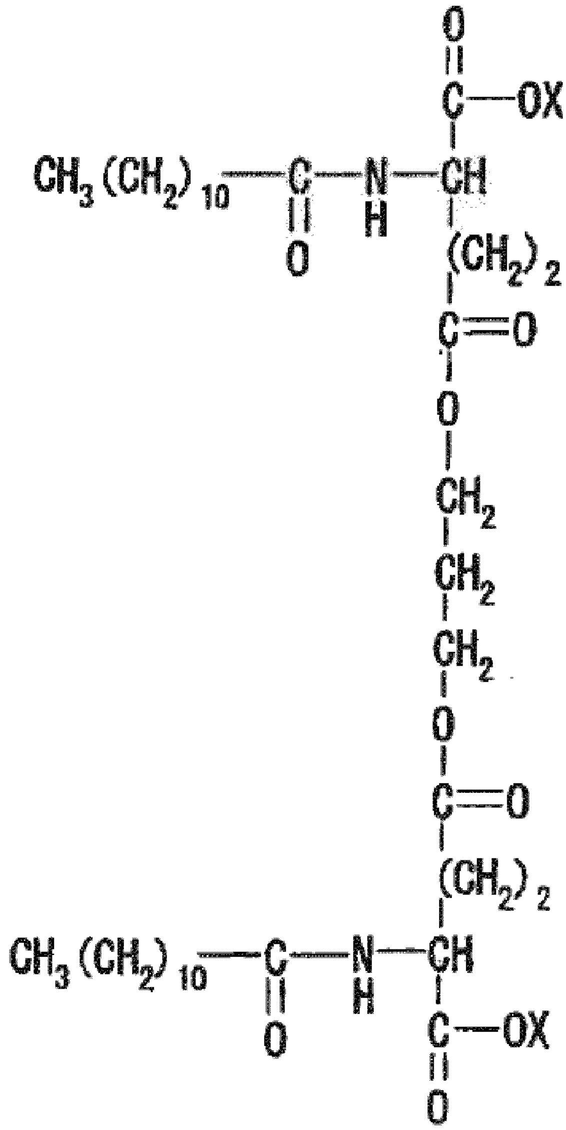

Verbindung b

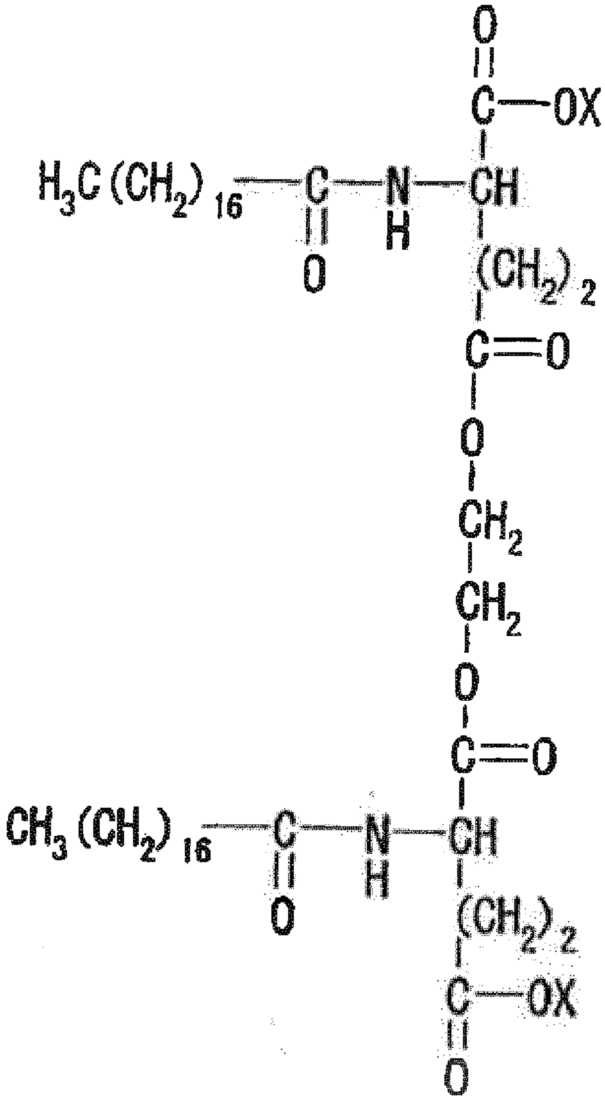

Verbindung c

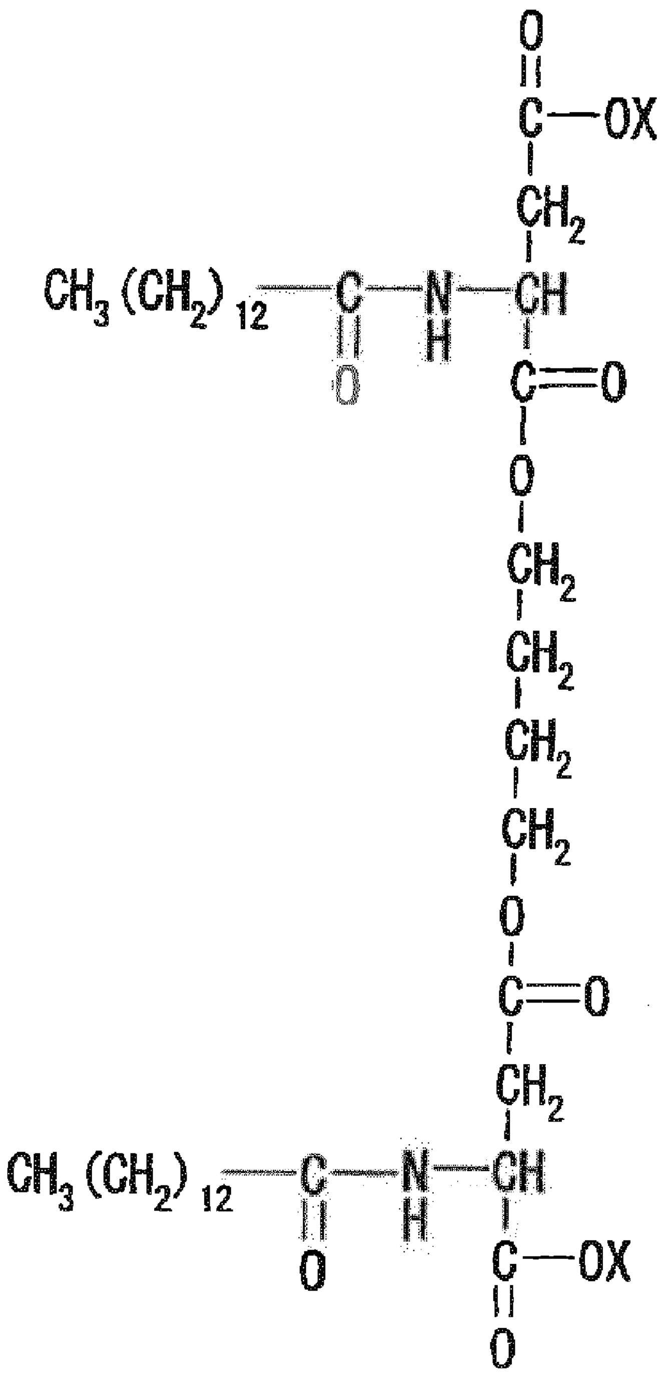

Verbindung d

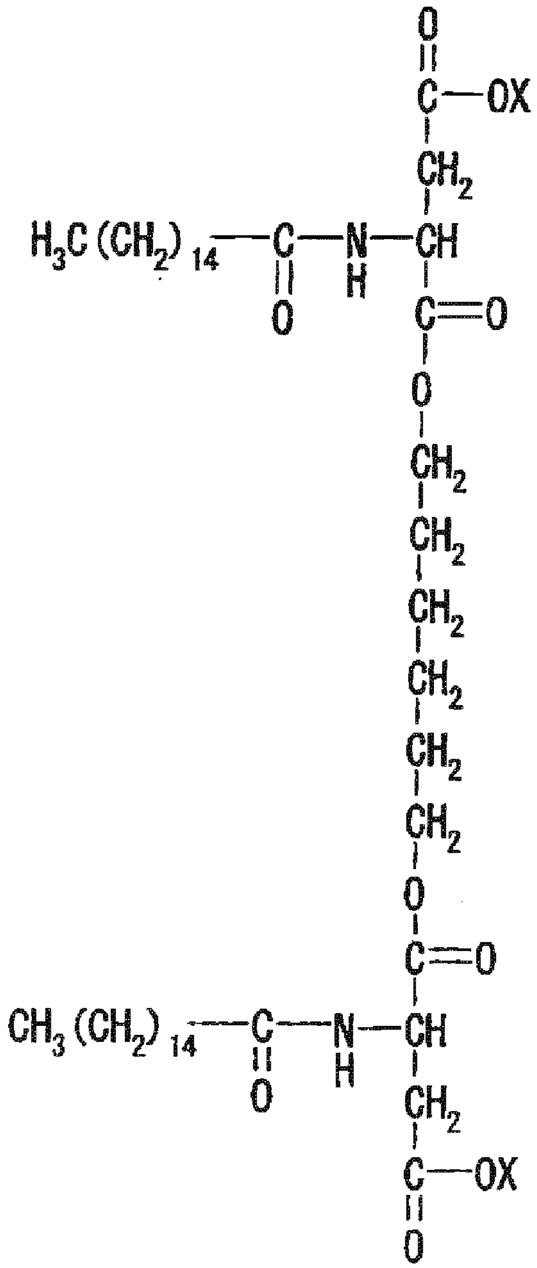

Verbindung e

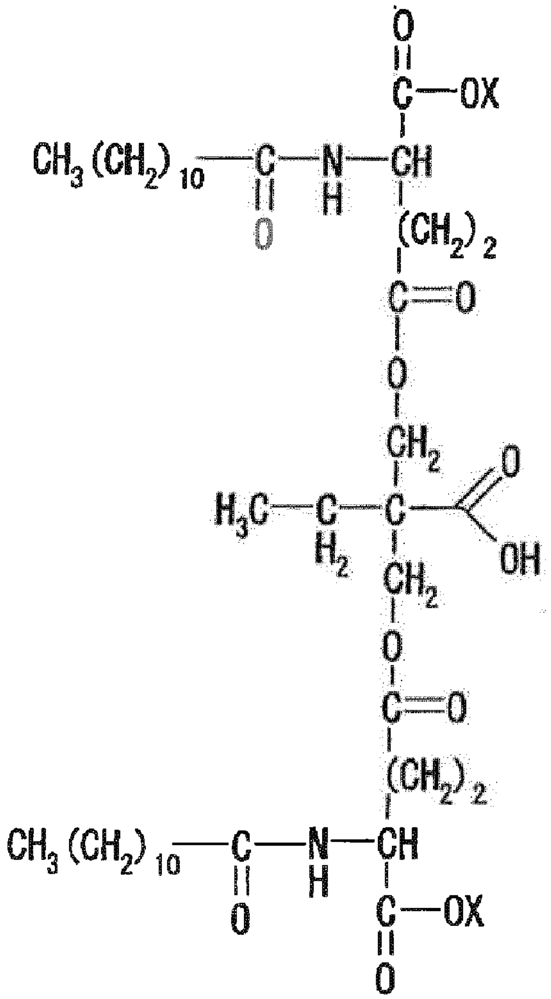

Verbindung f

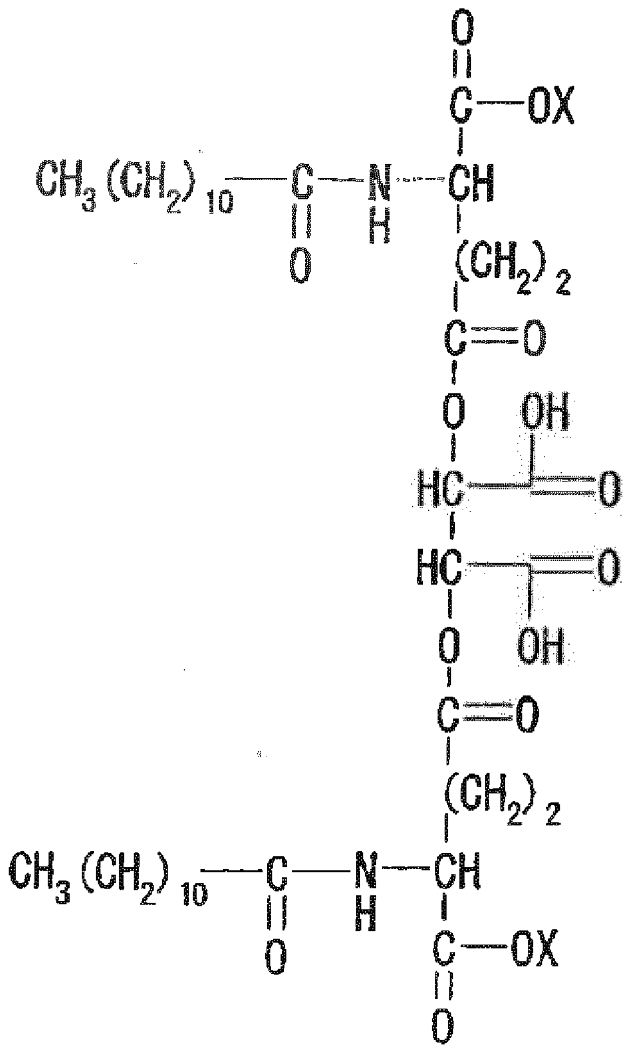

Verbindung g

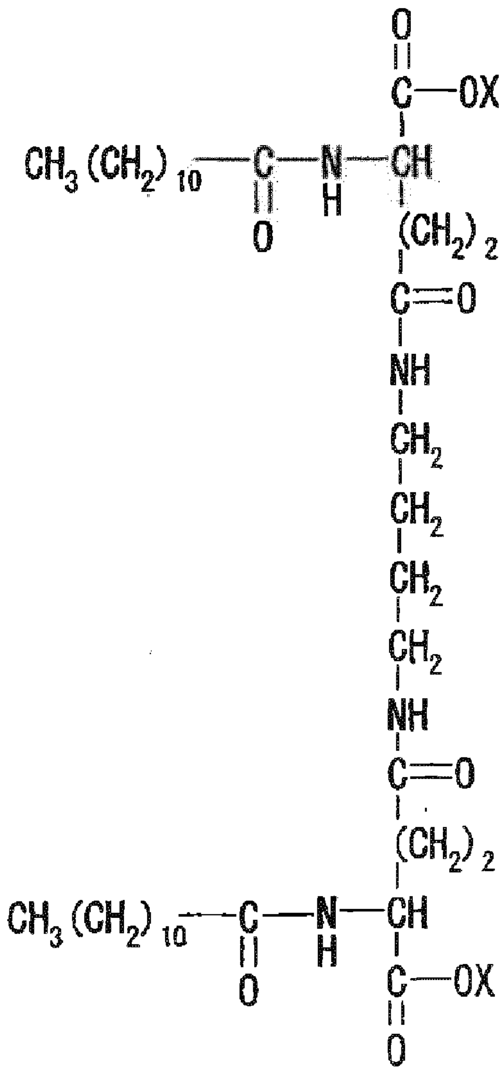

Verbindung h

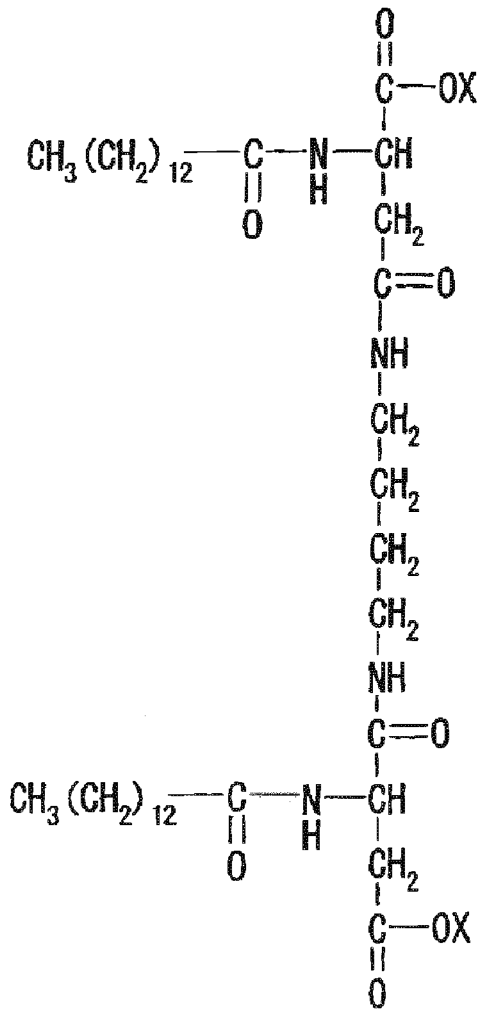

Verbindung i

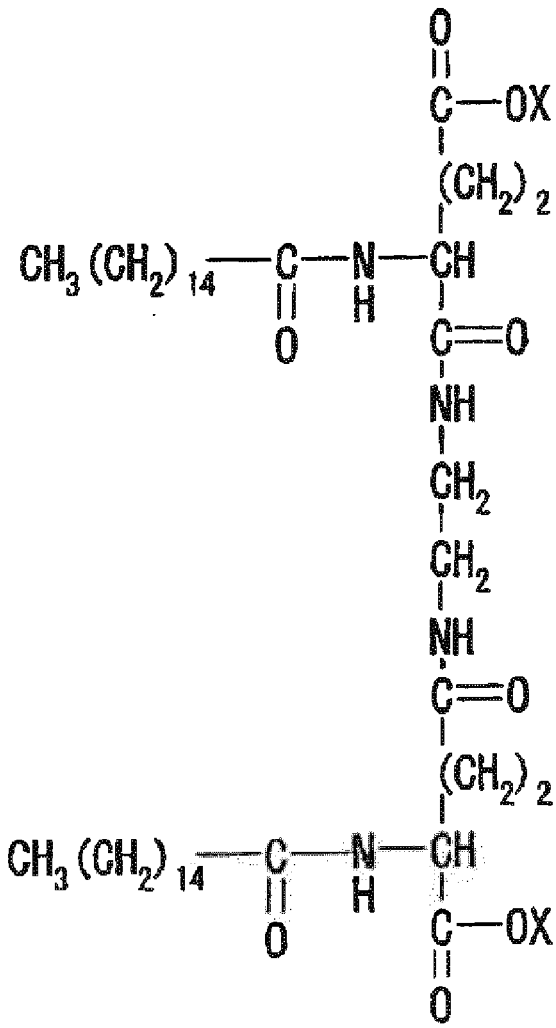

Verbindung j

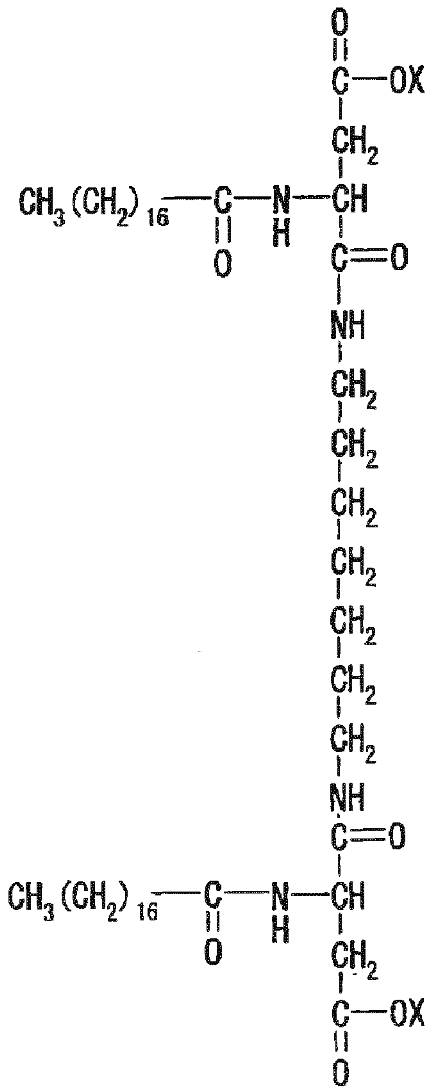

Verbindung k

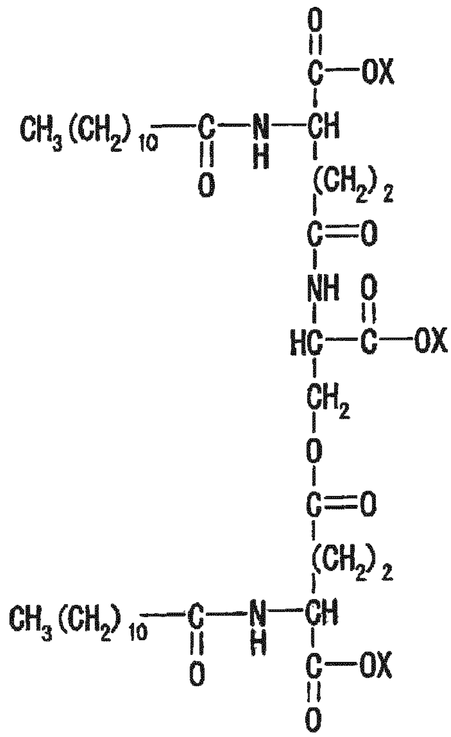

Verbindung l

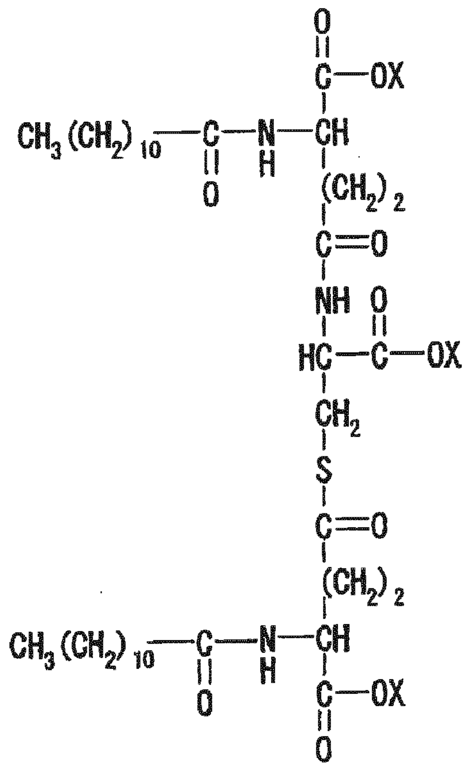

und unter Verbindungen (Gemischen) der folgenden Formeln (5) bis (7):

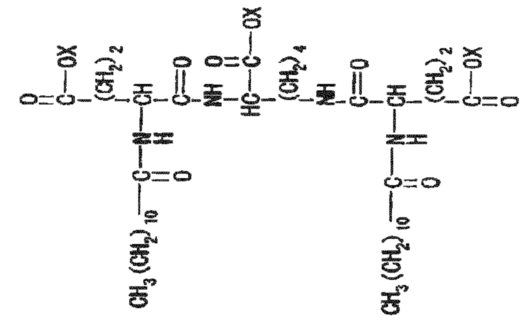 ....(5)

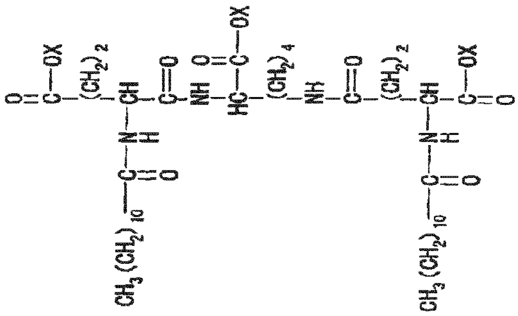

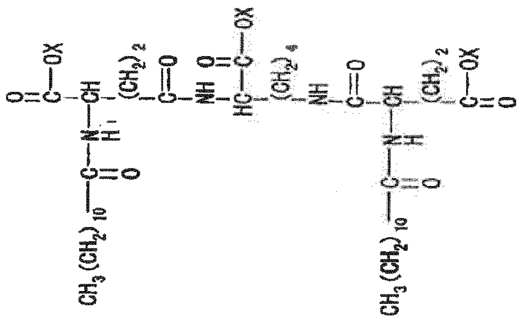

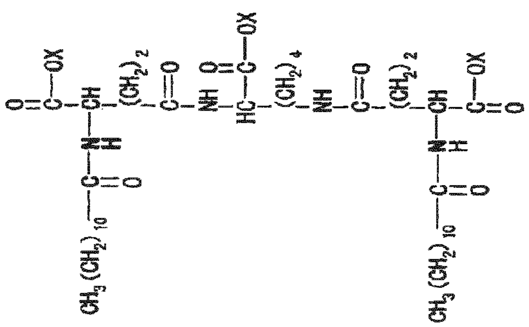

worin X unabhängig entweder H oder Na darstellt,

...(6)

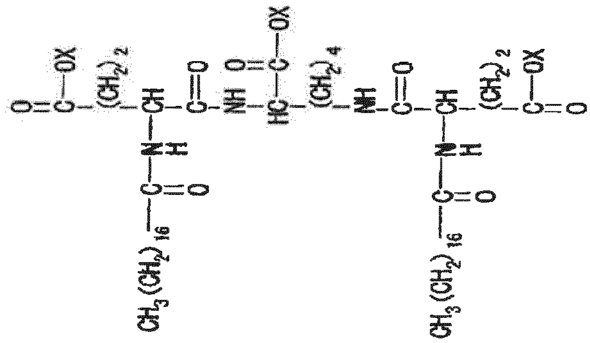

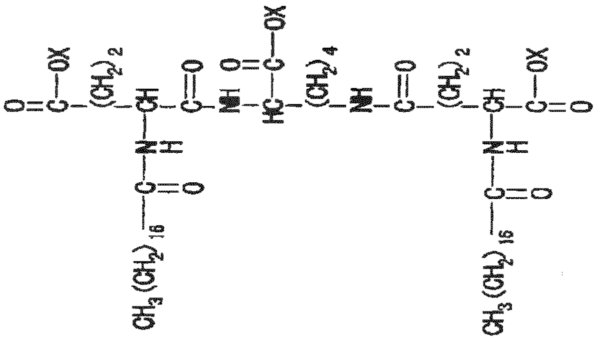

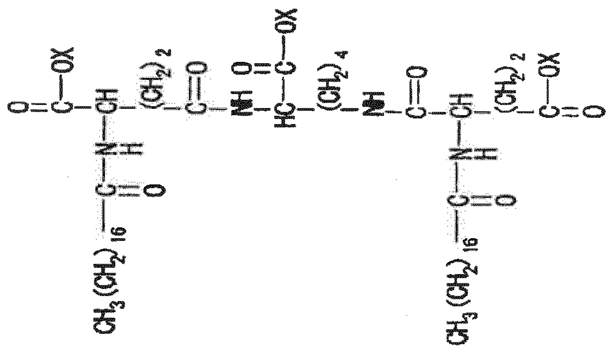

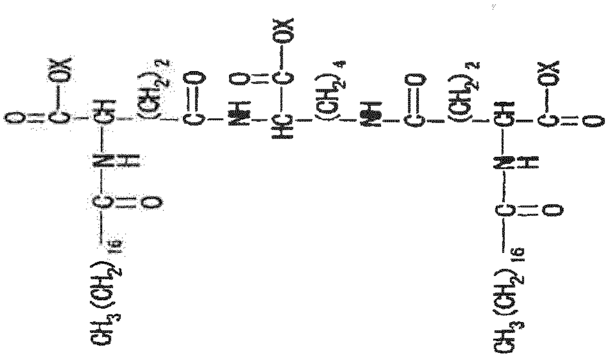

worin X unabhängig H oder Na darstellt,

...(7)

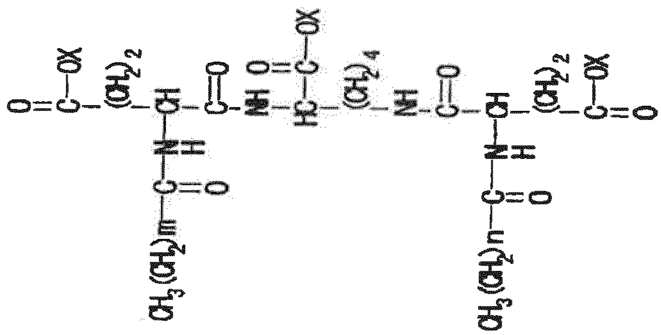

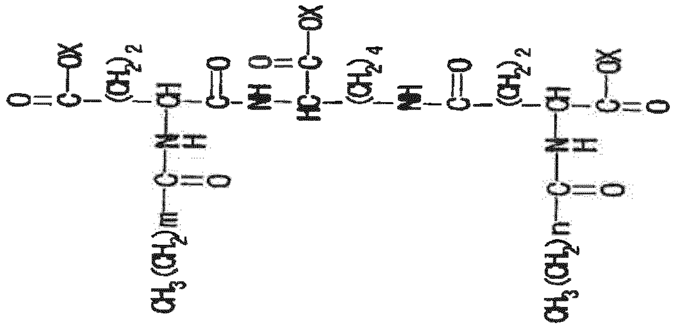

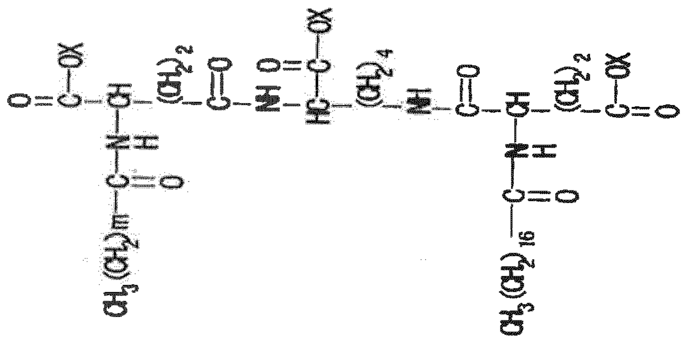

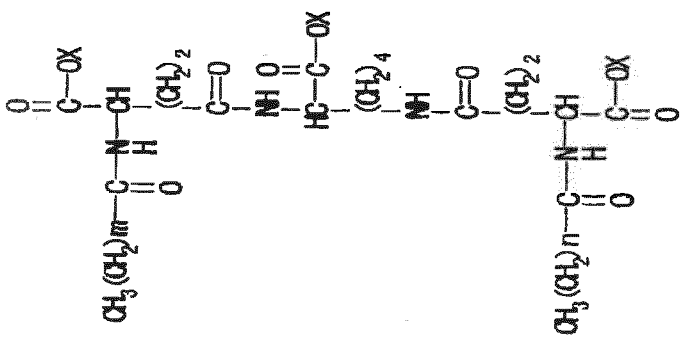

worin X unabhängig H oder Na darstellt und m und n unabhängig voneinander ganze Zahlen von 6 bis 16 darstellen, und (B) mindestens eine Art einer ölbasierten Komponente enthält, wobei die ölbasierte Komponente ein Flüssigkristallbildungshilfsmittel ist, das mindestens eine Art einer Sterolverbindung ist, wobei das Molverhältnis des Flüssigkristallbildungshilfsmittels zu (A) (Menge des Flüssigkristallbildungshilfsmittels : Menge von (A)) 1:10 oder höher und 10:1 oder niedriger ist.

2. Zusammensetzung nach Anspruch 1, wobei (A) Flüssigkristalle bildet.

3. Körperoberflächenschutzzusammensetzung nach Anspruch 1, wobei die Sterolverbindung mindestens eine ist, die aus der Gruppe ausgewählt ist, die aus Cholesterin, Cytosterol, Lanosterol, Phytosterol, Dihydrocholesterin, Dihydrolanosterol und Dehydrocholesterin und Estern davon besteht.

4. Körperoberflächenschutzzusammensetzung nach Anspruch 3, wobei das Molverhältnis des Flüssigkristallbildungsmittels zu (A) (Menge des Flüssigkristallbildungshilfsmittels : Menge von (A)) 1:5 oder höher und 5:1 oder niedriger ist.

5. Körperoberflächenschutzzusammensetzung nach einem der Ansprüche 1 bis 4, die außerdem (C) eine Hornzellinterzellulärkomponente und/oder (D) ein wasserlösliches Feuchtigkeitsmittel enthält.

6. Äußere Zubereitung zum Verringern oder Verhindern einer allergenen oder entzündlichen Hautkrankheit, welche die Körperoberflächenschutzzusammensetzung nach einem der Ansprüche 1 bis 5 enthält.

7. Kosmetisches Körperpflegemittelprodukt, das die Körperoberflächenschutzzusammensetzung nach einem der Ansprüche 1 bis 5 enthält.

8. Arzneimittelprodukt, das die Körperoberflächenschutzzusammensetzung nach einem der Ansprüche 1 bis 5 enthält.

9. Körperoberflächenschutzmittel, das die Körperoberflächenschutzzusammensetzung nach einem der Ansprüche 1 bis 5 enthält.

10. Verwendung der Körperoberflächenschutzzusammensetzung nach einem der Ansprüche 1 bis 5 zum Herstellen einer äußeren Zubereitung zum Reduzieren oder Verhindern einer allergenen oder entzündlichen Hautkrankheit.

11. Verwendung der Körperoberflächenschutzzusammensetzung nach einem der Ansprüche 1 bis 5, zum Herstellen eines kosmetischen Körperpflegemittlprodukts.

12. Verwendung der Körperoberflächenschutzzusammensetzung nach einem der Ansprüche 1 bis 5 zum Herstellen eines Arzneimittelprodukts.

13. Verwendung der Körperoberflächenschutzzusammensetzung nach einem der Ansprüche 1 bis 5 zum Herstellen eines Körperoberflächenschutzmittels.

## Revendications

1. Composition de protection de la surface d'un corps, comprenant, (A) au moins un type de composé qui est un composé à plusieurs chaînes de type groupes hydrophiles multiples choisi parmi les composés a à I ci-dessous :

composé a

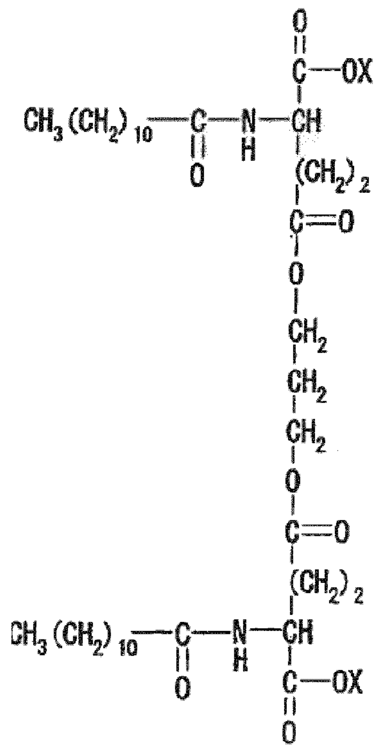

composé b

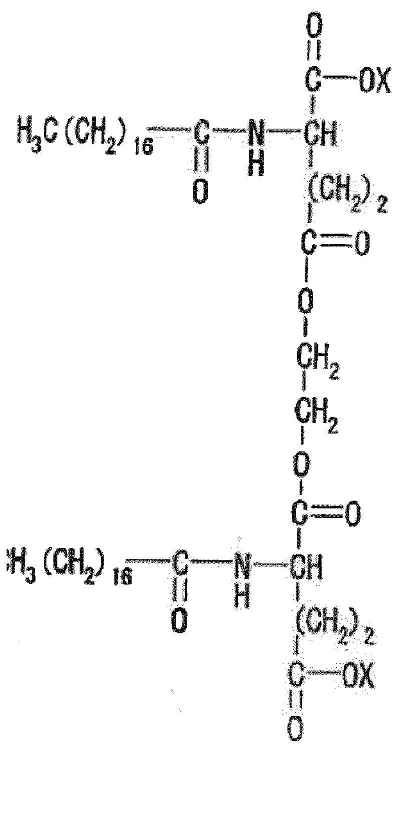

composé c

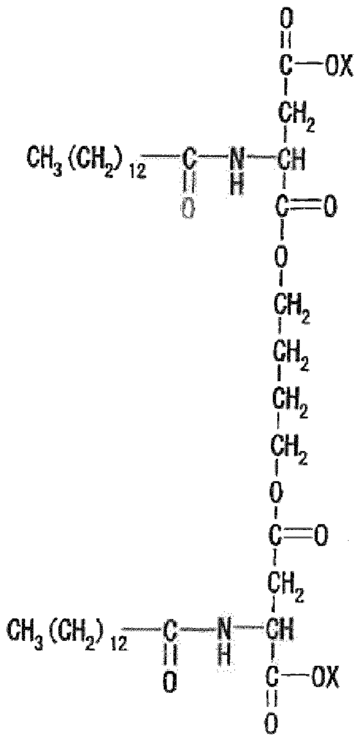

composé d

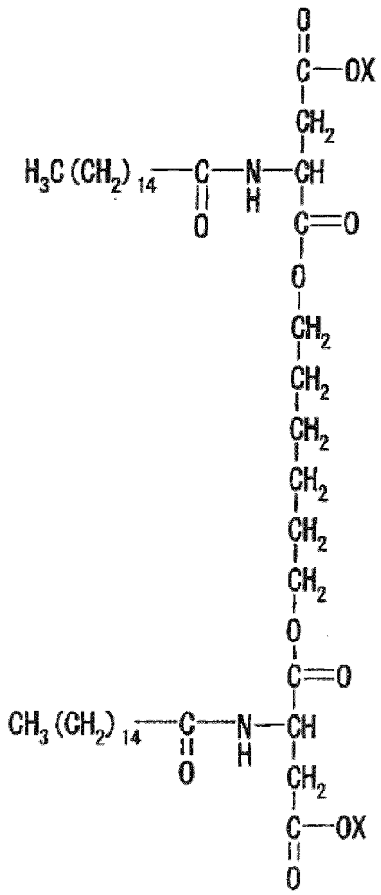

composé e

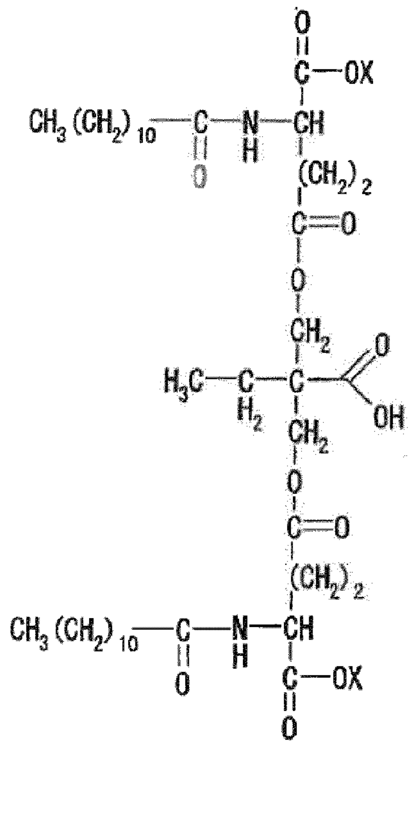

composé f

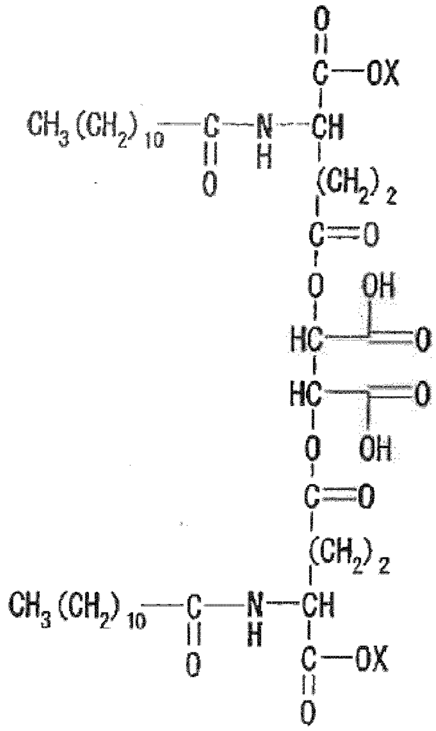

composé g

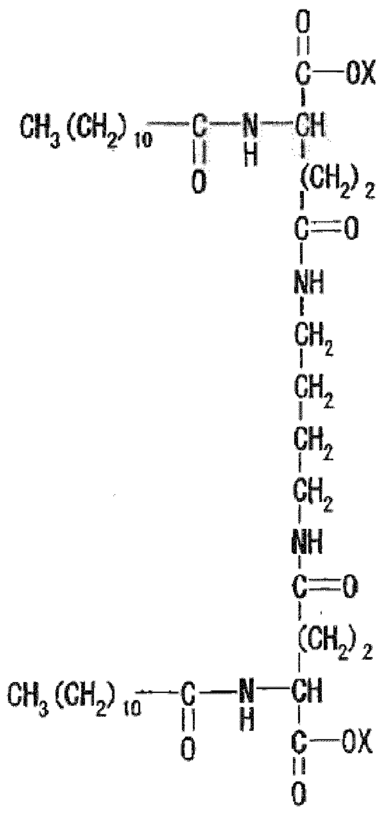

composé h

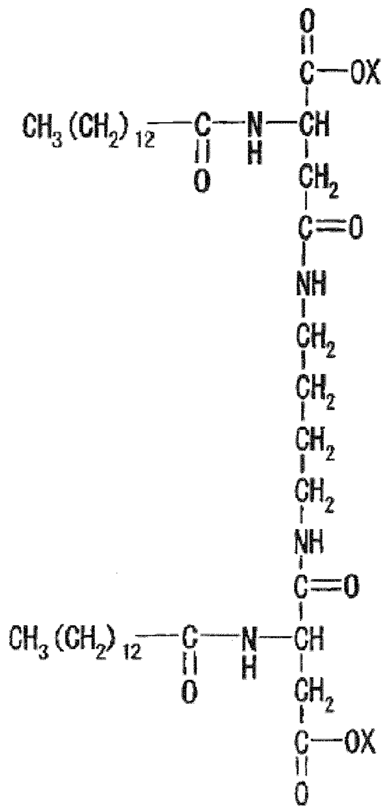

composé i

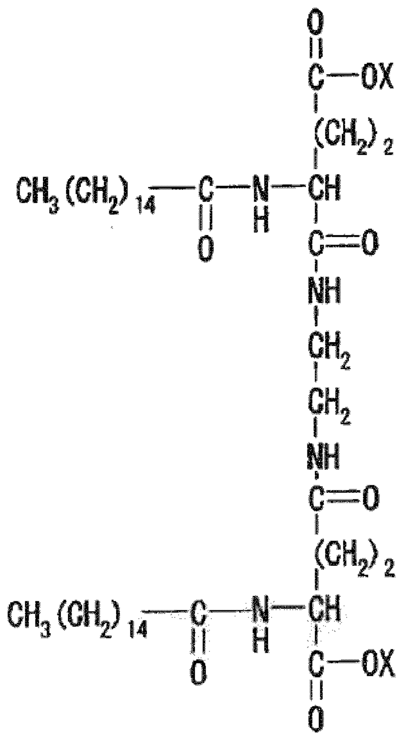

composé j

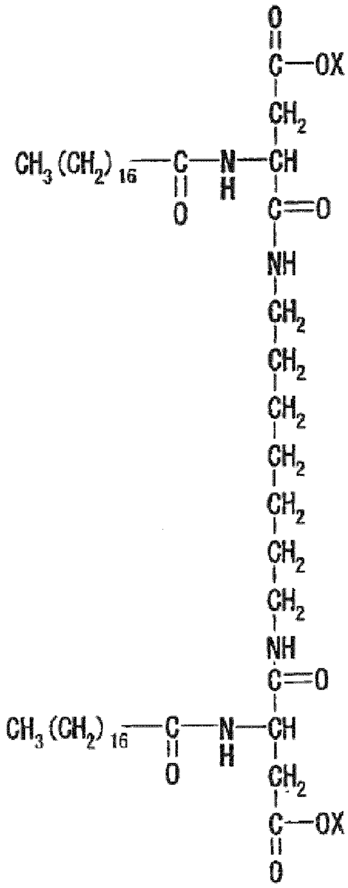

composé k

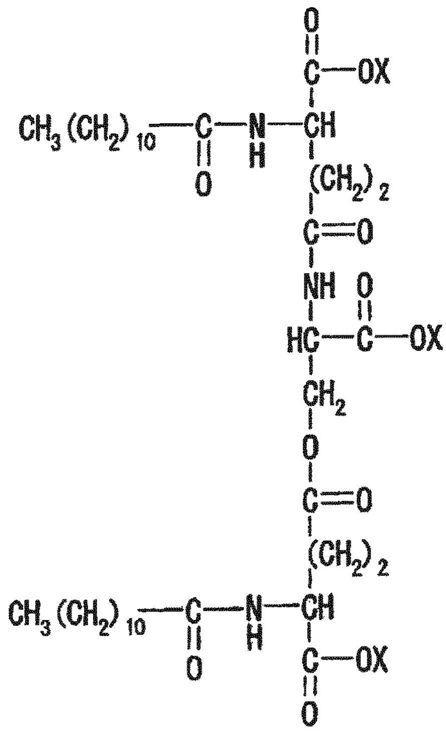

composé I

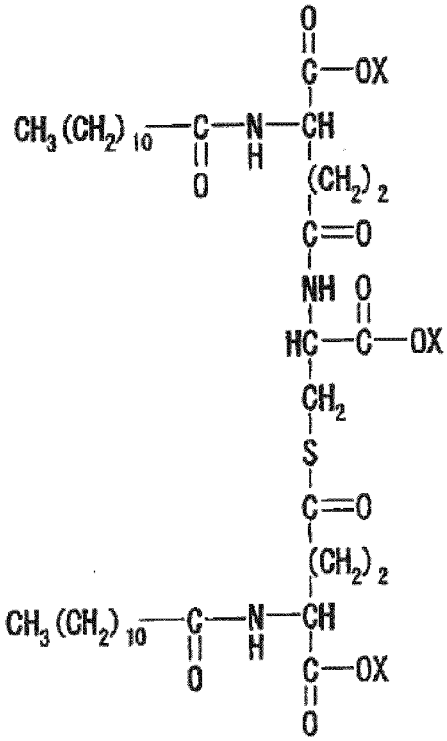

et les composés (mélanges) représentés par les formules (5) à (7) suivantes :

....(5)

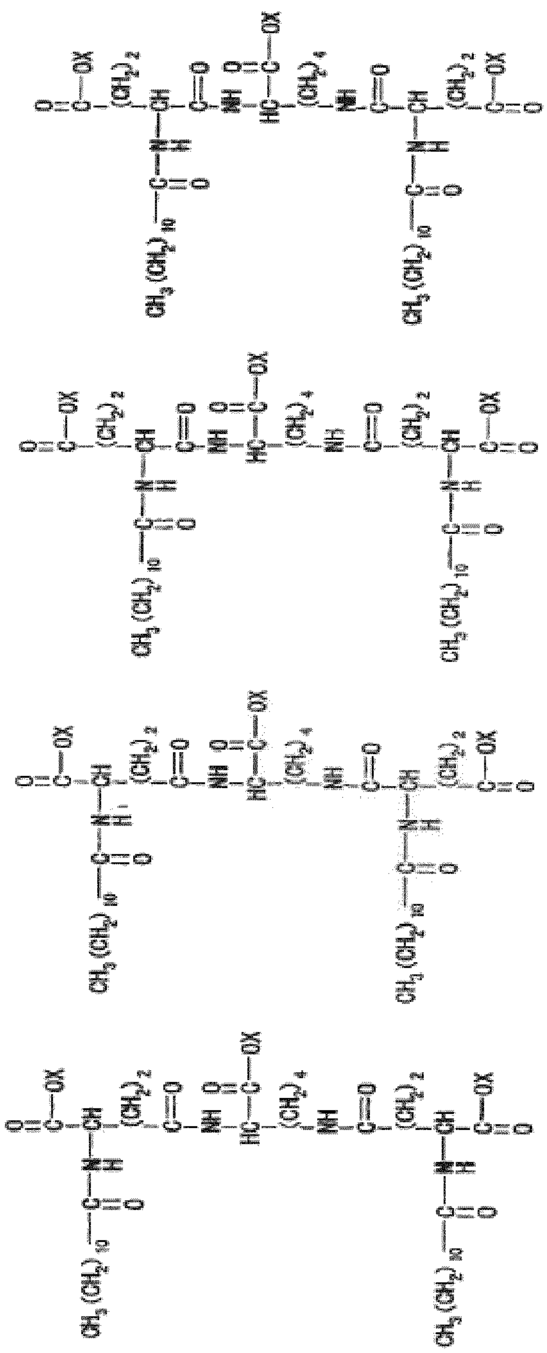

formule dans laquelle X représente indépendamment un atome d'H ou de Na,

•••(6)

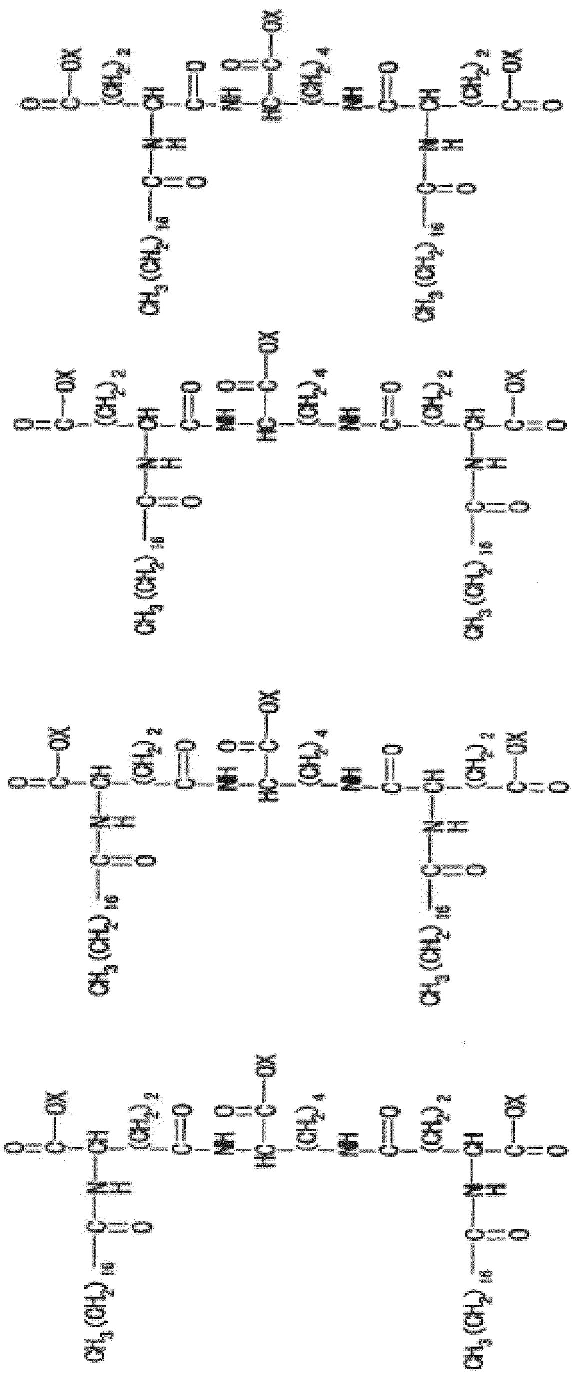

formule dans laquelle X représente indépendamment un atome d'H ou de Na,

...(7)

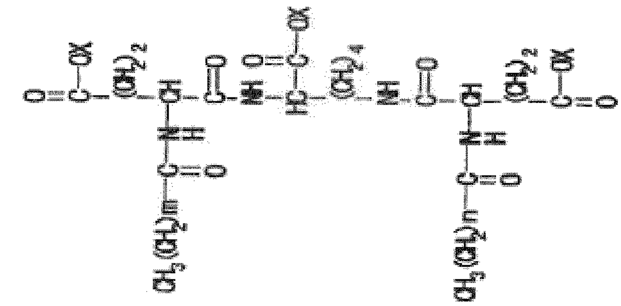

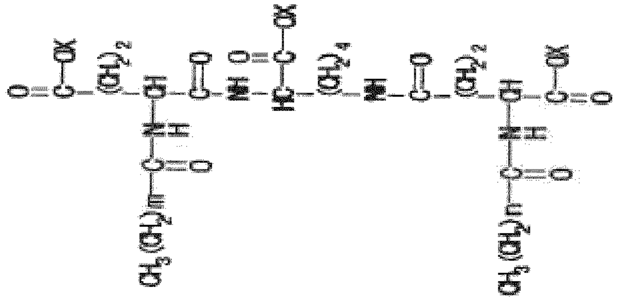

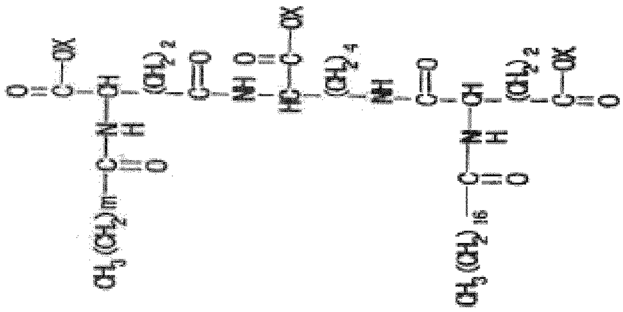

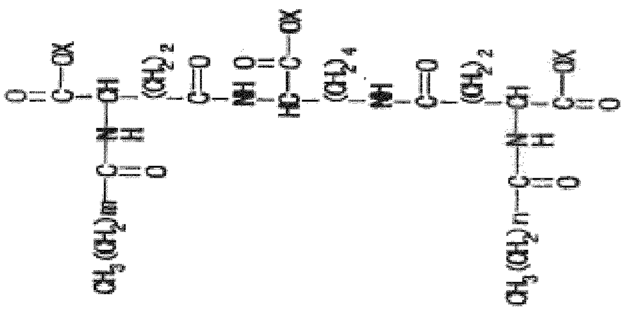

formule dans laquelle X représente indépendamment un atome d'H ou de Na, et m et n représentent chacun indépendamment des nombres entiers de 6 à 16
et (B) au moins un type de composant à base d'huile, ledit composant à base d'huile étant un auxiliaire de formation de cristaux liquides qui est au moins un type de composé de stérol, le rapport molaire dudit auxiliaire de formation de cristaux liquides à (A) (quantité d'auxiliaire de formation de cristaux liquides/quantité de (A)) étant de 1/10 ou supérieur à 10/1 ou inférieur.

**2.** Composition selon la revendication 1, dans laquelle ledit (A) forme des cristaux liquides.

**3.** Composition de protection de la surface d'un corps selon la revendication 1, dans laquelle ledit composé de stérol

est au moins un composé choisi dans un groupe constitué du cholestérol, du cytostérol, du lanostérol, du phytostérol, du dihydrocholestérol, du dihydrolanostérol et du déshydrocholestérol et de leurs esters.

4. Composition de protection de la surface d'un corps selon la revendication 3, dans laquelle le rapport molaire dudit auxiliaire de formation de cristaux liquides à (A) (quantité d'auxiliaire de formation de cristaux liquides/quantité de (A)) est de 1/5 ou supérieur à 5/1 ou inférieur.

5. Composition de protection de la surface d'un corps selon l'une quelconque des revendications 1 à 4, comprenant en outre (C) un composant intercellulaire de la couche cornée et/ou (D) un agent hydratant soluble dans l'eau.

6. Préparation externe permettant de réduire ou de prévenir une maladie de peau de type allergique ou inflammatoire, comprenant la composition de protection de la surface d'un corps selon l'une quelconque des revendications 1 à 5.

7. Produit cosmétique de toilette comprenant la composition de protection de la surface d'un corps selon l'une quelconque des revendications 1 à 5.

8. Produit pharmaceutique comprenant la composition de protection de la surface d'un corps selon l'une quelconque des revendications 1 à 5.

9. Agent de protection de la surface d'un corps comprenant la composition de protection de la surface d'un corps selon l'une quelconque des revendications 1 à 5.

10. Utilisation de la composition de protection de la surface d'un corps selon l'une quelconque des revendications 1 à 5 pour la fabrication d'une préparation externe permettant de réduire ou de prévenir une maladie de peau de type allergique ou inflammatoire.

11. Utilisation de la composition de protection de la surface d'un corps selon l'une quelconque des revendications 1 à 5 pour la fabrication d'un produit cosmétique de toilette.

12. Utilisation de la composition de protection de la surface d'un corps selon l'une quelconque des revendications 1 à 5 pour la fabrication d'un produit pharmaceutique.

13. Utilisation de la composition de protection de la surface d'un corps selon l'une quelconque des revendications 1 à 5 pour la fabrication d'un agent de protection de la surface d'un corps.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000191499 A
- JP H10218754 B
- JP H10203956 B
- JP H10219278 B
- WO 200539642 A
- JP H9124432 B
- JP H5213731 B
- JP 2001039859 A
- JP 2002167313 A
- JP 2004168735 A
- EP 1547998 A1
- WO 2004020394 A1
- JP 2004168736 A
- JP 2004168675 A
- JP 2006045063 A
- JP 2006045064 A
- JP 2006056874 A